# EUROPÄISCHE PATENTANMELDUNG

(11) **EP 4 403 196 A1**
(43) Veröffentlichungstag der Anmeldung: **24.07.2024**
(21) Anmeldenummer: 23152890.2
(22) Anmeldetag: 23.01.2023
(51) Int. Cl.: A61L 27/26, A61L 27/36, A61L 27/38, A61L 27/52, B33Y 70/00

(54) **HERZSCHRITTMACHER-KONSTRUKT, VERFAHREN ZUR HERSTELLUNG EINES HERZSCHRITTMACHER-KONSTRUKTS UND DESSEN VERWENDUNG**

(71) Anmelder: Universität Rostock, 18055 Rostock (DE)
(72) Erfinder: Kussauer, Sophie, 18055 Rostock (DE); David, Robert, 18209 Wittenbeck (DE); Polley, Christian, 18055 Rostock (DE); Seitz, Hermann, 18055 Rostock (DE); Distler, Thomas, 90459 Nürnberg (DE); Boccaccini, Aldo R., 91054 Erlangen (DE)
(74) Vertreter: Altmann Stößel Dick Patentanwälte PartG mbB

(57) **Zusammenfassung**

Die Erfindung betrifft in einem ersten Aspekt ein Herzschrittmacher-Konstrukt umfassend eine dreidimensionale Kernstruktur umfassend Schrittmacherzellen und Fibroblasten, *in vitro* hergestellt oder *in vitro* herstellbar mittels eines additiven Fertigungsverfahrens und/oder eines Abformverfahrens. Ein zweiter Aspekt der Erfindung bezieht sich auf ein Verfahren zur Herstellung eines Herzschrittmacher-Konstrukts, insbesondere eines Herzschrittmacher-Konstrukt gemäß des ersten Aspekts der Erfindung. Gemäß eines dritten Aspektes betrifft die Erfindung ein Herzschrittmacher-Konstrukt, erhalten oder erhältlich nach dem Verfahren des zweiten Aspektes. Ein vierter Aspekt der Erfindung bezieht sich auf die Verwendung eines Herzschrittmacher-Konstrukts gemäß des ersten Aspektes oder eines Herzschrittmacher-Konstrukts nach dem dritten Aspekt, zur Untersuchung der Auswirkung von Stoffen, insbesondere Schadstoffen und/oder Medikamenten, auf die Funktion des sinoatrialen Knotens.

## Beschreibung

Die Erfindung betrifft in einem ersten Aspekt ein Herzschrittmacher-Konstrukt umfassend eine dreidimensionale Kernstruktur umfassend Schrittmacherzellen und Fibroblasten, *in vitro* hergestellt oder *in vitro* herstellbar mittels eines additiven Fertigungsverfahrens und/oder eines Abformverfahrens. Ein zweiter Aspekt der Erfindung bezieht sich auf ein Verfahren zur Herstellung eines Herzschrittmacher-Konstrukts, insbesondere eines Herzschrittmacher-Konstrukt gemäß des ersten Aspekts der Erfindung. Gemäß eines dritten Aspektes betrifft die Erfindung ein Herzschrittmacher-Konstrukt, erhalten oder erhältlich nach dem Verfahren des zweiten Aspektes. Ein vierter Aspekt der Erfindung bezieht sich auf die Verwendung eines Herzschrittmacher-Konstrukts gemäß des ersten Aspektes oder eines Herzschrittmacher-Konstrukts nach dem dritten Aspekt, zur Untersuchung der Auswirkung von Stoffen, insbesondere Schadstoffen und/oder Medikamenten, auf die Funktion des sinoatrialen Knotens.

Das Sick-Sinus-Syndrom (SSS) beschreibt verschiedene Krankheiten, die auf eine gestörte Funktion des sinoatrialen Knotens (SAN), dem natürlichen Herzschrittmacher, zurückzuführen sind. Die Größe des menschlichen SAN beträgt ungefähr 1,3 cm in der Länge und 0,5 cm in der Breite mit einer ovalen Gesamtform. Er besteht aus spezialisierten Kardiomyozyten, die von autonomen Nerven innerviert werden. Mittels 3D-Strukturanalyse konnte in den letzten Jahren eine funktionell beobachtete SAN-Grenze, die aus Fibrose, Fett und/oder diskontinuierlichen Fasern besteht zwischen SAN und Vorhöfen festgestellt werden. Diese Grenze scheint ungefähr 200 µm dick zu sein und wird nur von verzweigten Myofasertrakten in Bereichen der SAN-Erregungsleitungswege durchzogen. Die Erkrankungen des Sinusknotens haben in den letzten Jahren bei Erwachsenen dramatisch zugenommen und treten besonders bei Kindern mit angeborenen Herzfehlern auf. Diese jungen Patienten entwickeln häufig Rhythmusstörungen, die durch chirurgische oder interventionelle Therapien oder durch den Herzfehler selbst verursacht werden. Zu den Fehlfunktionen des kardialen Reizleitungssystems gehören Sinusbradykardie, SA-Block, Sinusarrest sowie das Tachykardie-Bradykardie-Syndrom. Das SSS ist gekennzeichnet durch eine Fibrose des Schrittmachergewebes und geht häufig mit anderen Erkrankungen wie ischämischen Herzerkrankungen, Kardiomyopathien oder Myokarditis einher. Dieser Zustand führt zu Fehlfunktion des Herzschrittmachers oder zu einer gestörten Weiterleitung der elektrischen Impulse vom Sinusknoten zum Vorhof, die zusammenfassend als "sinoatriale Erregungsleitungsstörungen" bezeichnet werden. Bislang beruhen die therapeutischen Ansätze bei SSS auf der Implantation künstlicher elektronischer Herzschrittmacher. Diese Geräte stimulieren das natürliche Reizleitungssystem, um eine korrekte Erregung des Herzmuskels zu ermöglichen. Kausale Therapieansätze, d. h. ein biologischer Ersatz des kranken Sinusknotens, sind derzeit nicht verfügbar. Außerdem sprechen künstliche Herzschrittmacher nicht auf autonome Stimulation an, die Implantation und Überwachung sind kostenintensiv und die Gefahr von Infektionen und Elektrodenversagen sind weitere limitierende Nachteile, um nur einige zu nennen. Auch wenn durch Optimierungen der elektronischen Schrittmacher dem hohen Risiko für schwere Komplikationen entgegengewirkt werden soll, besteht ein dringender Bedarf an innovativen regenerativen Therapieansätzen zur Behandlung von Herzrhythmusstörungen, insbesondere bei betroffenen Kindern.

Um diese Limitationen zu überwinden, wird seit vielen Jahren an der Generierung von biologischen Schrittmachern geforscht. WO 2020/028809 A1 beispielsweise beschreibt ein Scaffold eines biologischen Schrittmachers mit einer Isolationsschicht, welche allerdings nicht biologischen Ursprungs ist. Das grundlegende Gerüst des Schrittmachers ist aus einem polymeren Material wie beispielsweise Polystyrol gebildet, welches eine Beschichtung mit Schrittmacherzellen aufweist.

Die jüngsten Fortschritte wurden bei der gezielten Differenzierung von induzierten pluripotenten Stammzellen (iPSC) in Herzschrittmacherzellen erzielt, welche eine Chance für die Translation bieten.

Um biologische Schrittmacherzellen für eine zukünftige Therapie zu erhalten, gibt es unterschiedliche Ansätze: Ein Ansatz beruht auf der Transplantation von *in vitro* erzeugten biologischen Schrittmachern, die aus iPSC stammen. Die Verfügbarkeit solcher Zellen stellt aktuell noch ein Problem dar, da sie in der *in vitro* Generierung nicht mehr als etwa 10-20 % der Kardiomyozyten-Population ausmachen. In einer Reihe von Veröffentlichungen wurde die Erzeugung eines biologischen Herzschrittmachers durch einfache Zelltransplantation oder "direkte Reprogrammierung" von Herzmuskelzellen beschrieben, jedoch vernachlässigten diese Studien die komplexe 3D-Architektur des SAN. Dementsprechend waren die erzielten Erfolge begrenzt.

Noor et al. 2019 beschreiben sowohl das Bioprinting von vaskularisiertem und kontraktilem Herzgewebe als auch die Herstellung eines miniaturisierten menschlichen Herzens/ Herzkammer-ähnlicher Organoide. Diese Arbeiten zeigen jedoch nicht die Herstellung eines funktionsfähigen Herzens. Weiterhin müssen noch eine Reihe von Hürden überwunden werden, bevor ein solches Herzkonstrukt eingesetzt werden kann: Die Arbeit von Noor et al. ging nicht auf Kardiomyozyten-Subtypen ein, die sich sowohl in ihrem Phänotyp als auch in ihren physiologischen Eigenschaften unterscheiden. Diese müssen implizit an ihren richtigen Stellen lokalisiert ("gedruckt") werden, um eine echte Herzfunktion zu gewährleisten (d.h. Vorhof- vs. Kammer- vs. Schrittmacher-Kardiomyozyten usw.). Außerdem konnten bisher nur Herzkonstrukte mit einer entsprechenden Abmessung von nicht mehr als ca. 2 cm in der Länge und 1,5 cm im Durchmesser erreicht werden. Diese stellen nur eine Miniaturisierung des Herzens dar. Darüber hinaus wurde in weiteren Publikationen die Herstellung von Herzähnlichen Strukturen/Gewebepatches mittels Bioprinting beschrieben, die jedoch die unterschiedlichen Herzmuskelzellen sowie deren natürliche Lage und Funktion in keinerlei Weise berücksichtigen. Im Gegensatz zu bereits publizierten Versuchen unter Verwendung von isolierten oder iPSC-derivierten Herzmuskelzellen wurden bisher weder Herzschrittmacherzellen für das Bioprinting von funktionellen Organoiden verwendet, noch wurden, insbesondere per Bioprinting, SAN-gleiche oder SAN-ähnliche Konstrukte erzeugt.

Die Aufgabe der vorliegenden Erfindung war daher die Bereitstellung eines biologischen Schrittmachers mit SAN-gleicher oder -ähnlicher Struktur.

### 1. Aspekt - Herzschrittmacher-Konstrukt

Gemäß eines ersten Aspektes betrifft die Erfindung ein Herzschrittmacher-Konstrukt umfassend eine dreidimensionale Kernstruktur umfassend Schrittmacherzellen und Fibroblasten, *in vitro* hergestellt oder *in vitro* herstellbar mittels eines additiven Fertigungsverfahrens und/oder eines Abformverfahrens.

Die Schrittmacherzellen und Fibroblasten liegen bevorzugt als Gemisch vor, d.h. insbesondere nicht in räumlich voneinander abgegrenzten Bereichen o.ä., sondern Schrittmacherzellen und Fibroblasten sind miteinander vermischt. Ein "Herzschrittmacher-Konstrukt" ist eine *in vitro* erzeugte Version eines biologischen Herzschrittmachers, insbesondere eines sinoatrialen Knotens, das *in vitro* in drei Dimensionen hergestellt wurde und eine realistische Anatomie aufweist.

In bevorzugten Ausführungsformen des Herzschrittmacher-Konstrukts enthält die Kernstruktur weiterhin Teile der extrazellularen Matrix der Fibroblasten. Teile der extrazellulären Matrix der Fibroblasten sind ausgewählt aus fibrillärem Kollagen, bevorzugt ein oder mehrere ausgewählt aus der Gruppe bestehend aus Kollagen I (Col I), Kollagen II (Col II) und Kollagen III (Col III), Elastin, und Basement-Membran Protein, bevorzugt ein oder mehrere Basement-Membran Protein(e) ausgewählt aus der Gruppe bestehend aus Col IV, Fibronectin und Laminin. Weiter bevorzugt ist mindestens ein fibrilläres Kollagen, weiter bevorzugt mindestens Col I, enthalten.

In bevorzugten Ausführungsformen des Herzschrittmacher-Konstrukts sind innerhalb der Kernstruktur keine nicht-leitfähigen Polymere (wie beispielsweise Polystyrol und/oder Parylen) vorhanden, bevorzugt sind innerhalb der Kernstruktur keine Stützelemente aus einem oder mehreren Material(ien) ausgewählt aus der Gruppe von nicht-leitfähigem Kunststoff, Glas, Metall und Metalllegierung, weiter bevorzugt keine Stützelemente nicht-biologischen Ursprungs, vorhanden, wobei weiter bevorzugt generell keine weiteren Stützelemente vorhanden sind.

Im Hinblick auf beispielsweise die WO 2020/028809 A1 ist anzumerken, dass Polymere wie beispielsweise Polystyrol und Parylene nicht elektrisch leitend sind, vielmehr werden sie aufgrund ihrer isolierenden Eigenschaften oft als (elektrische) Isolatoren verwendet.

In bevorzugten Ausführungsformen des Herzschrittmacher-Konstrukts weist die Kernstruktur ein zellbasiertes Verhältnis von Schrittmacherzellen zu Fibroblasten im Bereich von 10:1bis 1:10 auf. Weiterhin weist die Kernstruktur bevorzugt eine Größe (Volumen) im Bereich von 5 bis 500 mm³, bevorzugt im Bereich von 50 bis 250 mm³ auf.

In bevorzugten Ausführungsformen des Herzschrittmacher-Konstrukts umfasst dieses weiterhin eine Isolationsschicht (fibrotische Schicht) umfassend Adipozyten und Fibroblasten, angeordnet auf der Oberfläche der Kernstruktur, welche die Kernstruktur zu mindestens 90%, bevorzugt zu mindestens 95%, flächendeckend umgibt, wobei ein erster lokal begrenzter Bereich der Oberfläche der Kernstruktur unbedeckt ist, welcher als Signalübertragungsstelle dient oder dienen kann und ein zweiter lokal begrenzter Bereich der Oberfläche der Kernstruktur unbedeckt ist, welcher dem Anschluss bzw. dem Durchtritt eines Blutgefäßes, insbesondere einer Arterie, dient oder dienen kann. Weiter bevorzugt ist die gesamte Oberfläche der Kernstruktur mit Ausnahme des ersten und zweiten lokal begrenzten Bereichs bedeckt.

In bevorzugten Ausführungsformen des Herzschrittmacher-Konstrukts weist die Isolationsschicht eine Dicke im Bereich von 150 bis 250 µm, bevorzugt im Bereich von 100 bis 300 µm, weiter bevorzugt im Bereich von 50 bis 500 µm, weiter bevorzugt im Bereich von 50 bis 1000 µm, auf.

In bevorzugten Ausführungsformen des Herzschrittmacher-Konstrukts umfasst dieses weiterhin ein Blutgefäß, welches an dem zweiten lokal begrenzten unbedeckten Bereich der Oberfläche der Kernstruktur angeordnet oder anordenbar ist.

In bevorzugten Ausführungsformen des Herzschrittmacher-Konstrukts ist das additive Fertigungsverfahren ein dreidimensionales additives Fertigungsverfahren, weiter bevorzugt ein extrusionsbasiertes Biodruck-Verfahren, ein Drop-on-Demand Biodruck-Verfahren (insbesondere ein Tintenstrahl-Verfahren), ein lithografisches Verfahren, ein mikrofluidisches Biodruck-Verfahren, ein Laser-unterstütztes Biodruck-Verfahren, oder eine Mischung von zwei oder mehr dieser Verfahren, wobei das dreidimensionale additive Fertigungsverfahren bevorzugt ein extrusionsbasiertes Biodruck-Verfahren oder ein lithografisches Verfahren, weiter bevorzugt ein extrusionsbasiertes Biodruck-Verfahren, ist; und wobei das Abformverfahren bevorzugt ein Spritzgussverfahren oder ein Mikrospritzgussverfahren ist.

In bevorzugten Ausführungsformen des Herzschrittmacher-Konstrukts wird das Gemisch aus Schrittmacherzellen und Fibroblasten suspendiert in einem Hydrogel aus oxidierter Alginat-Gelatine eingesetzt, woraus, bevorzugt mittels extrusionsbasiertem Biodruck-Verfahren, eine dreidimensionale Struktur (Scaffold) erzeugt wird.

In bevorzugten Ausführungsformen des Herzschrittmacher-Konstrukts wird die dreidimensionale Struktur, optional mit aufgebrachter Isolationsschicht, kultiviert, bevorzugt bei einer Temperatur im Bereich von 32 bis 41°C, weiter bevorzugt in einem flüssigen Medium, für einen Zeitraum von mindestens einem Tag, bevorzugt mindestens 3 Tagen, weiter bevorzugt mindestens 7 Tagen, unter Erhalt der Kernstruktur aus dreidimensional angeordneten Schrittmacherzellen und Fibroblasten, welche optional von der Isolationsschicht umgeben ist. Das flüssige Medium ist bevorzugt ein Zellkultur-Medium, weiter bevorzugt ein Schrittmacherzellmedium, welches auf alle vorhandenen Zelltypen angepasst ist. Geeignete Medien und deren Anpassung sind dem Fachmann bekannt.

In bevorzugten Ausführungsformen des Herzschrittmacher-Konstrukts umfasst dieses weiterhin ein Mikrokanalnetzwerk zumindest innerhalb der Kernstruktur aus dreidimensional angeordneten Schrittmacherzellen und Fibroblasten, wobei die Wandung der Kanäle des Mikrokanalnetzwerks bevorzugt aus Endothelzellen gebildet ist und weiter bevorzugt die Kanäle des Mikrokanalnetzwerks einen inneren Durchmesser im Bereich von 100-500 µm aufweisen. Das Mikrokanalnetzwerk selbst hat Durchtritte durch die Isolationsschicht um den venösen Abfluss des Blutes zu gewährleisten. Es ist gebildet durch Abzweigungen aus dem großen Blutgefäß, welche innerhalb der Kernstruktur ausgebreitet sind.

In bevorzugten Ausführungsformen des Herzschrittmacher-Konstrukts wird das Mikrokanalnetzwerk durch für das Bioprinting der dreidimensionalen Struktur eingesetzte Opfertinten erzeugt oder ist durch diese erzeugbar oder das Mikrokanalnetzwerk wird, insbesondere beim FRESH-Verfahren, durch Druck in ein (thermo)reversibles Stützbad (support bath) aus einem Stützmaterial, insbesondere Gelatine, bevorzugt aus passend aufbereiteter mikropartikulärer Gelatine, erzeugt. Entsprechende Stützbäder bzw. die dafür passend aufbereiteten mikropartikulären Gelatinen sind dem Fachmann bekannt (beispielsweise Hinton et al. Science Advances 2015, Fresh 2.0; Lee et al. Science 2019 mit Pluronic F127 & Gum Arabic; Bliley et al. Biofabrication 2022) oder Poloxamer/PEG (Colly et al. Langmuire 2021). "Opfertinte" bzw. "Stützmaterial" meint ein Material, welches nur über einen begrenzten Zeitraum im Scaffold verbleibt, um dort als Support oder Stützstruktur zu dienen und ohne Rückstände herausgelöst werden kann, wobei das Herauslösen beispielsweise mittels Lösungsmitteleinsatz oder mittels Temperaturänderung erfolgt - entsprechend bedeutet "(thermo)reversibel, dass das jeweilige Material durch geeignete Änderung der Temperatur herauslösbar ist und somit Hohlräume erzeugt werden (siehe Mikrokanalnetzwerk und Vaskularisation); die Opfertinte ist ebenfalls bevorzugt (thermo)reversibel. Beispielhaft zu nennen als Opfertinte bzw. Stützmaterial ist Gelatine. "Poloxamer" ist ein (Block-co)Polymer aus mindestens einem C2 bis C5 Alkylenoxid, bevorzugt ein Blockcopolymer aus Ethylenoxid und Propylenoxid, "Pluronic F127" ist ein Triblock-copolymer Poly(ethylenoxid)-poly(propylenoxid)-poly(ethylen oxid (PEO-PPO-PEO, CAS Nummer: 9003-11-6). Die Opfertinten werden bei extrusionsbasierten Biodruckverfahren getrennt von der Biotinte gedruckt.

In bevorzugten Ausführungsformen des Herzschrittmacher-Konstrukts besteht die Kernstruktur zu mindestens 90 Gewichts-%, bevorzugt zu mindestens 95 Gewichts-%, weiter bevorzugt zu mindestens 99 Gewichts-% aus Schrittmacherzellen und Fibroblasten und optional Teilen der extrazellularen Matrix der Fibroblasten, sowie optional Endothelzellen, jeweils bezogen auf ein Gesamtgewicht der Kernstruktur von 100 Gewichts-%.

In bevorzugten Ausführungsformen des Herzschrittmacher-Konstrukts sind die Schrittmacherzellen *in vitro* generierte Schrittmacherzellen, insbesondere *in vitro* generierte humane Schrittmacherzellen. Diese können mittels verschiedener Verfahren *in vitro* aus Stammzellen generiert werden, wobei das Verfahren bevorzugt ausgewählt ist aus Umprogrammierung, direkter Programmierung, und Mischformen von zwei oder mehr dieser Verfahren.

Gemäß einer bevorzugten Ausführungsform des Herzschrittmacher-Konstrukts sind die *in vitro* generierten Schrittmacherzellen aus Myokardzellen, bevorzugt mittels Umprogrammierung, erzeugt. Die direkte Umprogrammierung kann beispielsweise aus ventrikulären Kardiomyozyten, gegebenenfalls unter Verwendung von Tbx-Faktoren wie beispielsweise Tbx3 oder Tbx18, erfolgen. Exemplarisch ist eine entsprechende Umprogrammierung in Beispiel 1 der WO 2013/070952 A1 offenbart, deren Offenbarung hier durch Bezugnahme integriert ist.

In einer Ausführungsform des Herzschrittmacher-Konstrukts sind die *in vitro* generierten Schrittmacherzellen aus embryonalen Stammzellen, bevorzugt mittels direkter Programmierung, weiter bevorzugt mittels direkter Programmierung unter Einsatz von Shox2, erzeugt. Eine entsprechende direkte Programmierung ist beispielsweise in Beispiel 4 der WO 2013/070952 A1 offenbart, welches hier durch Bezugnahme integriert ist.

In einer Ausführungsform des Herzschrittmacher-Konstrukts sind die *in vitro* generierten Schrittmacherzellen aus humanen multipotenten Stammzellen oder tierischen multipotenten Stammzellen oder humanen nicht-embryonalen pluripotenten Stammzellen oder tierischen embryonalen pluripotenten Stammzellen oder tierischen nicht-embryonalen pluripotenten Stammzellen oder tierischen parthenogenetischen pluripotenten Stammzellen oder humanen parthenogenetischen pluripotenten Stammzellen oder tierischen spermatogonialen pluripotenten Stammzellen oder humanen spermatogonialen pluripotenten Stammzellen, weiter bevorzugt aus humanen multipotenten Stammzellen oder tierischen multipotenten Stammzellen oder humanen nicht-embryonalen pluripotenten Stammzellen oder tierischen embryonalen pluripotenten Stammzellen oder tierischen nicht-embryonalen pluripotenten Stammzellen oder tierischen parthenogenetischen pluripotenten Stammzellen oder tierischen spermatogonialen pluripotenten Stammzellen oder humanen spermatogonialen pluripotenten Stammzellen, bevorzugt mittels direkter Programmierung, erzeugt (induzierte sinuatriale Zellkörperchen (iSABs) umfassend Herz-Schrittmacherzellen). Bevorzugt werden hierbei Sinusknotenzellen (Herz-Schrittmacherzellen) aus Stammzellen erzeugt, bei welchem eine Nukleinsäure in Stammzellen eingebracht wird, wodurch diese einen Tbx-Transkriptionsfaktor oder Shox2 exprimieren, oder ein Tbx-Protein in die Stammzellen eingebracht wird, wobei zusätzlich ein Konstrukt zur Expression eines Antibiotikum-Resistenz-Gens, welches durch einen alpha-MHC (MYH6)-Promoter gesteuert wird, eingebracht wird und die resultierenden Stammzellen in Gegenwart des Antibiotikums differenziert werden. Beschrieben ist diese Erzeugung von Sinusknotenzellen in der WO 2015/091157 A1, deren Offenbarung hier durch Bezugnahme aufgenommen ist. Insbesondere ist in dieser Ausführungsform das in vitro generierte Herzschrittmachergewebe umfassend Herz-Schrittmacherzellen aus den oben beschriebenen Stammzellen, erzeugt, wobei mindestens ein Tbx-Transkriptionsfaktor, insbesondere Tbx3, in Kombination mit einer Antibiotikaselektion auf der Basis des Myh6-Promotors eingesetzt wird. Wenn eine Nukleinsäure zur Expression eines Tbx-Transkriptionsfaktors in die Stammzellen eingebracht wird, wird diese vorzugsweise ausgewählt aus Tbx-DNS, insbesondere Tbx-cDNS; oder Tbx-RNS, insbesondere Tbx-mRNS. Im Rahmen der RNS können Tbx-mRNS in die Stammzellen transfiziert werden, wobei dies keine stabile Gen-Modifikation ergibt. Alternativ können mikro-RNS eingebracht werden, die endogenes Tbx zur Expression bringen. In einer bevorzugten Ausführungsform der Nukleinsäure-Einbringung wird Tbx-DNS, insbesondere Tbx-cDNS mittels Vektor, insbesondere mittels (Über-)Expressionsvektor, eingebracht. Das Tbx ist vorzugsweise ausgewählt aus Tbx3 oder Tbx18, wobei Tbx3 besonders bevorzugt und Tbx3-cDNS höchst bevorzugt ist. D.h. eine höchst bevorzugte Variante ist die Einbringung von Tbx3-cDNS mit Überexpressionsvektor. Hinsichtlich des Tbx-Proteins, welches ebenfalls keine (stabile) Gen-Modifikation verursacht, ist ebenfalls Tbx3 bevorzugt. Eingesetzt werden humane oder nicht-humane Nukleinsäuren oder Proteine, wobei diejenigen humaner Herkunft bevorzugt sind. Im Rahmen der Erfindung werden in einigen Ausführungsformen nichtvirale Episomen / Episomale Vektoren eingesetzt. Dies sind ringförmige, extrachromosomale DNA Strukturen, mit Replikationsursprung und chromosomaler Haftfrequenz. Sie finden Anwendung als kostengünstige, Transgen- und virenfreie Reprogrammierungsmethode von iPS-Zellen oder verschiedener somatischer Zelltypen (Flexibilität). Das Einbringen der Vektoren erfolgt durch Elektroporation oder Transfektion der Zellen, die anschließende extrachromosomale Replikation geht ohne feste Integration ins Wirtsgenom vonstatten. Somit besteht kein Risiko von Störungen im Genom oder unvorhersehbaren Integrationsartefakten. Episomale Vektoren werden an die Tochterzellen weitergegeben, es erfolgt kein Abbau des Vektors, aber bei dieser Replikationsrate (nur einmal pro Zellzyklus) gehen die Episome mit einer Rate von etwa 5% pro Zellgeneration verloren. Als autonome Einheit werden die Episomen über das "Huckepack Prinzip" (J. Bode et al. (2001) an die Tochterzellen ungezielt übertragen. Neben den oben bereits genannten episomalen Vektoren Tbx3, Tbx18 können auch Shox2 oder ein pEBNA-basiertesTbx3-Expressionskonstrukt (Invitrogen GeneArt) (A. Baiker et al. (2000)). Ebenso können im Rahmen der vorliegenden Erfindung modifizierte mRNSn eingesetzt werden: Protein-kodierende Messenger RNSs tragen die Informationen zum Aufbau eines spezifischen Proteins und verfügen somit über ein hohes Potenzial in der Zell-(Re-)Programmierung. Aufgrund von Eigenschaften wie einer hohen Instabilität, einer potenziellen Immunogenität und fehlenden effektiven Einbringungsmethoden sind Modifikationen notwendig, die diese Eigenschaften verbessern und dadurch in einer optimierten Anwendbarkeit resultieren. Beispielhaft genannt werden können hier translationsfördernde Veränderungen am 5'-Cap oder modifizierte Nukleoside. Die Tatsache, dass theoretisch jede gewünschte mRNS in vitro hergestellt werden kann, um die Biosynthese von Zielproteinen in jedem beliebigen Zelltyp zu induzieren birgt ein breites Spektrum von Anwendungsmöglichkeiten. Weitere Vorteile sind, dass mRNSn biologischen Ursprungs sind, einen sofortigen, aber nur vorübergehender Wirkungsmechanismus haben und dabei nicht ins Wirtsgenom integrieren und wieder abgebaut werden, welches die Sicherheit in der Anwendung erhöht. Ihre Verwendung für die Zell -(Re-) Programmierung zielt auf die Generierung von induzierten Pluripotenten Stammzellen z.B. aus Fibroblasten ab oder via direktem Forward-programming von beispielweise Kardiomyozyten aus Fibroblasten. Auch als Differenzierungs-fördernde Faktoren hin zu Endothelzellen oder Myoblasten werden sie eingesetzt. Auch hier sind beispielhaft zu nennen Tbx3, Tbx18, Shox2 (O. Chabanovska et al. (2021)).

Die Antibiotikaselektion auf der Basis des Myh6-Promotors nutzt vorzugsweise ein Antibiotikum-Resistenz-Gen ausgewählt aus Aminoglykosid-Antibiotikum-Resistenz-Gen, mehr bevorzugt aus Neomycin- und Puromycin-Resistenz-Gen, höchst bevorzugt Neomycin-Resistenz-Gen. Das für die Selektion eingesetzte Antibiotikum ist entsprechend ausgewählt aus Aminoglykosid-Antibiotikum, insbesondere aus Neomycin und Puromycin. "Entsprechend ausgewählt" bedeutet, dass immer das zum Resistenz-Gen passende Antibiotikum eingesetzt wird, beispielsweise beim Neomycin-Resistenz-Gen wird anschließend mit Neomycin selektiert.
*In vitro* erzeugt und entsprechend eingesetzt werden in jedem Fall Herz-Schrittmacherzellen (human oder nicht human), wobei humane Herz-Schrittmacherzellen bevorzugt sind. Hierfür werden humane Stammzellen *in vitro* mit vorzugsweise humanem Protein oder humaner Nukleinsäure kombiniert. Kreuzkombinationen, wie beispielsweise die Einführung humaner Proteine oder humaner Nukleinsäure in nicht-humane, z.B. murine, Stammzellen ist ebenfalls möglich, auch die reine Kombination der nicht-humanen Vertreter zur Erzeugung nicht humaner Herz-Schrittmacherzellen.

Generell sind im Rahmen der vorliegenden Erfindung unterschiedliche Selektionsschritte, insbesondere während der zellulären Differenzierung, einsetzbar. Diese lassen sich untergliedern in genetische Modifikationen (z.B. die oben beschriebene Antibiotikaselektion, beispielsweise mittels Neomycin Resistenz) und nicht genetische Selektionsmethoden. Für letztere eignet sich bei Kardiomyozyten-Subtypen die Laktatselektion. Sie stellt eine Form der metabolischen Selektion dar und dient somit der Aufreinigung der Kardiomyozyten. Diese können im Vergleich zu anderen Zelltypen auch auf Laktat oder Fettsäuren als Substrat für die Energiegewinnung zurückgreifen. Dieser biochemische Unterschied zwischen Glukose- und Laktatmetabolismus bietet die Möglichkeit, die differenzierenden Kardiomyozyten von den nicht-Kardiomyozyten zu selektieren. Dies erfolgt durch eine Kultivierung in Glukose-freiem Medium unter Zugabe von Glutamat und Natriumlaktat. Den Nicht-Kardiomyozyten fehlt hierbei das/die passende Substrat/Energiequelle und sie sterben ab (S. Tohyama et al. 2013)).
Hinsichtlich der eingesetzten Stammzellen wie oben bereits beschrieben humanen multipotenten Stammzellen oder tierischen multipotenten Stammzellen oder humanen nicht-embryonalen pluripotenten Stammzellen oder tierischen embryonalen pluripotenten Stammzellen oder tierischen nicht-embryonalen pluripotenten Stammzellen oder tierischen parthenogenetischen pluripotenten Stammzellen oder humanen parthenogenetischen pluripotenten Stammzellen oder tierischen spermatogonialen pluripotenten Stammzellen oder humanen spermatogonialen pluripotenten Stammzellen, weiter bevorzugt aus humanen multipotenten Stammzellen oder tierischen multipotenten Stammzellen oder humanen nicht-embryonalen pluripotenten Stammzellen oder tierischen embryonalen pluripotenten Stammzellen oder tierischen nicht-embryonalen pluripotenten Stammzellen oder tierischen parthenogenetischen pluripotenten Stammzellen oder tierischen spermatogonialen pluripotenten Stammzellen oder humanen spermatogonialen pluripotenten Stammzellen. Humane embryonale Stammzellen sind in dieser bevorzugten sowie besonders bevorzugten Variante explizit ausgenommen.

In einer alternativen Ausführungsform werden keine ausdifferenzierten Stammzellen, sondern deren Vorläuferzellen für das Herzschrittmacher-Konstrukt bzw. für dessen Herstellungsverfahren verwendet. In dieser alternativen Ausführungsform werden als Vorläuferzellen bevorzugt Myokardzellen oder humane multipotente Stammzellen oder tierische multipotente Stammzellen oder humane nicht-embryonale pluripotente Stammzellen oder tierische embryonale pluripotente Stammzellen oder tierische nicht-embryonale pluripotente Stammzellen oder tierische parthenogenetische pluripotente Stammzellen oder humane parthenogenetische pluripotente Stammzellen oder tierische spermatogoniale pluripotente Stammzellen oder humane spermatogoniale pluripotente Stammzellen verwendet. Die Ausdifferenzierung erfolgt dann im ausgeformten Herzschrittmacher-Konstrukt. In einer Variante dieser alternativen Ausführungsform werden embryonale Stammzellen als Vorläuferzellen eingesetzt, wobei auch hier die Ausdifferenzierung dann im ausgeformten Herzschrittmacher-Konstrukt erfolgt.

In bevorzugten Ausführungsformen des Herzschrittmacher-Konstrukts sind die Fibroblasten humane Fibroblasten, weiter bevorzugt humane kardiale Fibroblasten, weiter bevorzugt humane kardiale Sinusknoten-Fibroblasten. Isolierte primäre humane kardiale Fibroblasten können käuflich erworben werden oder werden aus nicht-embryonalen pluripotenten Stammzellen differenziert. Spezifische Differenzierungsprotokolle sind dem Fachmann bekannt, beispielsweise aus J. Zhang et al. (2019).

In bevorzugten Ausführungsformen des Herzschrittmacher-Konstrukts sind die Adipozyten humane Adipozyten. Adipozyten werden differenziert aus isolierten humanen MSCs oder werden aus nicht-embryonalen pluripotenten Stammzellen differenziert, wobei das entsprechende Vorgehen dem Fachmann bekannt ist, beispielsweise aus Aghadi et al. (2022), Ahfeldt et al. (2012), Karam et al. (2020), oder Mohsen-Kanson et al. (2014).

Das erfindungsgemäße Herzschrittmacher-Konstrukt ist ausschließlich *ex vivo* bzw. *in vitro* erzeugt. Alle Schritte in der Erzeugung des Herzschrittmacher-Konstrukts erfolgen entsprechend ausschließlich *ex vivo* bzw. *in vitro.*

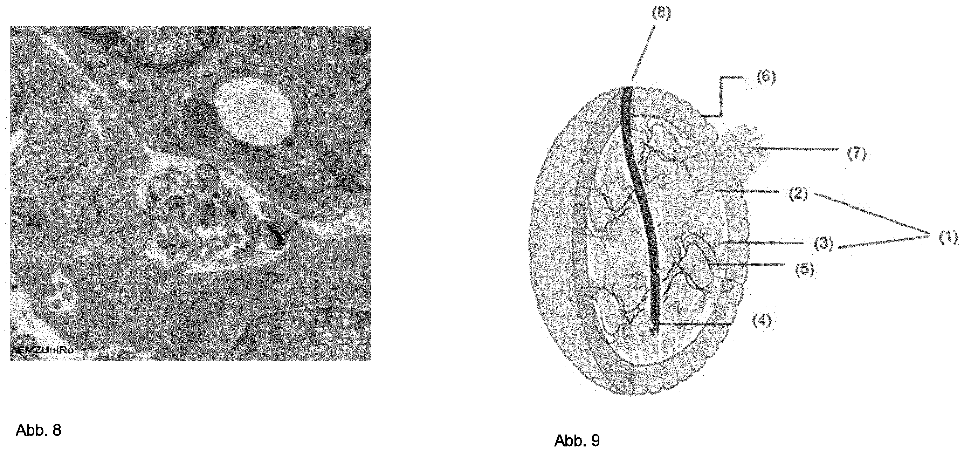

Ein erfindungsgemäße Herzschrittmacher-Konstrukt ist schematisch in Abb. 9 gezeigt: Das Herzschrittmacher-Konstrukt umfasst eine Kernstruktur (1), welche Schrittmacherzellen (2) und Fibroblasten (3), sowie Teile der extrazellularen Matrix der Fibroblasten umfasst. Innerhalb der Kernstruktur finden sich ein zentrales Blutgefäß (4) und ein Mikrokanalnetzwerk (5). Das Mikrokanalnetzwerk (5) weist dabei Durchtritte durch die Isolationsschicht (6) auf und ist gebildet durch Abzweigungen aus dem großen Blutgefäß (4), welche innerhalb der Kernstruktur (1) ausgebreitet sind. Die Kernstruktur (1) ist, zumindest anteilig, von einer Isolationsschicht (6) bedeckt und es existiert eine Signalübertragungsstelle (7), d.h. ein erster lokal begrenzter Bereich der Oberfläche der Kernstruktur ist unbedeckt. Weiterhin ist die Kernstruktur auch in einem zweiten lokal begrenzten Bereich der Oberfläche der Kernstruktur unbedeckt, welcher dem Anschluss bzw. dem Durchtritt des Blutgefäßes (8), insbesondere einer Arterie, dient oder dienen kann.

### 2. Aspekt - Verfahren zur Herstellung eines Herzschrittmacher-Konstrukts

Ein zweiter Aspekt der Erfindung betrifft ein Verfahren zur Herstellung eines Herzschrittmacher-Konstrukts, insbesondere eines Herzschrittmacher-Konstrukts gemäß des ersten Aspektes, umfassend:
(a) Bereitstellen einer Mischung aus Schrittmacherzellen und Fibroblasten;
(b) Erzeugen einer dreidimensionalen Struktur aus der gemäß (a) bereitgestellten Mischung und optional Opfertinte oder mittels eines (thermo)reversiblen Stützbades aus einem Stützmaterial mittels eines artifiziellen Fertigungsverfahrens und/oder eines Abformverfahrens.

"Artifiziell" meint ein künstliches, insbesondere ein technisches, Fertigungs- und/oder Abformverfahren.

Alle zum ersten Aspekt der Erfindung beschriebenen Details, Ausführungsformen und bevorzugten Ausführungsformen gelten gleichfalls für das Verfahren zur Herstellung eines Herzschrittmacher-Konstrukts des zweiten Aspektes.

Die gemäß (a) bereitgestellte Mischung enthält neben Schrittmacherzellen und Fibroblasten optional weitere Komponenten. Beispielsweise werden die Schrittmacherzellen und Fibroblasten suspendiert in einem Hydrogel aus oxidierter Alginat-Gelatine eingesetzt, bevorzugt wenn mittels extrusionsbasiertem Biodruck-Verfahren eine dreidimensionale Struktur (Scaffold) erzeugt wird.

In bevorzugten Ausführungsformen des Verfahrens zur Herstellung eines Herzschrittmacher-Konstrukts umfassen die Schritte (a) und (b):
(a.1) Bereitstellen einer Mischung aus Schrittmacherzellen und Fibroblasten;
(a.2) Bereitstellen von Endothelzellen;
(b) Erzeugen einer dreidimensionalen Struktur aus der gemäß (a.1) bereitgestellten Mischung und optional Opfertinte oder einem (thermo)reversiblen Stützbad aus einem Stützmaterial mit Erzeugen eines Mikrokanalnetzwerkes umfassend Endothelzellen aus den gemäß (a.2) bereitgestellten Endothelzellen, wobei das Mikrokanalnetzwerk innerhalb der dreidimensionalen Struktur erzeugt wird, jeweils mittels eines artifiziellen Fertigungsverfahrens.

In bevorzugten Ausführungsformen des Verfahrens zur Herstellung eines Herzschrittmacher-Konstrukts umfasst dieses weiterhin
(c) Aufbringen einer Isolationsschicht (fibrotische Schicht) umfassend Adipozyten und Fibroblasten auf die Oberfläche der gemäß (b) erzeugten dreidimensionalen Struktur mittels eines artifiziellen Fertigungsverfahrens, wobei mindestens 90%, bevorzugt mindestens 95%, der Oberfläche der gemäß (b) erzeugten dreidimensionalen Struktur bedeckt werden, wobei bevorzugt ein erster lokal begrenzter Bereich der Oberfläche der dreidimensionalen Struktur und optional ein zweiter lokal begrenzter Bereich der Oberfläche der dreidimensionalen Struktur unbedeckt gehalten wird, wobei der erste lokal begrenzte, unbedeckte Bereich der Oberfläche der dreidimensionalen Struktur als Signalübertragungsstelle angelegt ist und der optionale zweite lokal begrenzte, unbedeckte Bereich der Oberfläche als Durchtrittsstelle für eine Blutgefäß, insbesondere einer Arterie, angelegt ist;
unter Erhalt einer isolierend beschichteten dreidimensionalen Struktur.

In bevorzugten Ausführungsformen des Verfahrens zur Herstellung eines Herzschrittmacher-Konstrukts erfolgen die Schritte (b) und (c) nacheinander oder gleichzeitig.

Bei einer gleichzeitigen Durchführung der Schritte (b) und (c) wird bevorzugt mit mehreren Extrudern und/oder Mischungen, Einzelkomponenten, bevorzugt in Form sogenannter "Biotinten" in einem Setup gearbeitet. Eine "Biotinte" umfasst Zellen und zumindest ein Polymer, welches bevorzugt ausgewählt ist aus der Gruppe bestehend aus Polysaccharid, Protein, Glycosaminoglycan, Hyaluronsäure, Polyester und Mischungen von zwei oder mehr dieser Polymere, bevorzugt aus der Gruppe bestehend aus marinem Polysaccharid, Protein tierischen Ursprungs, rekombinant hergestelltem Protein, Hyaluronsäure, und Mischungen von zwei oder mehr dieser Polymere, weiter bevorzugt aus der Gruppe bestehend aus Alginat, Gellan Gum, Ulvan, Hyaluronsäure, Polyester und Mischungen von zwei oder mehr dieser Polymere, weiter bevorzugt aus der Gruppe bestehend aus Alginat, oxidiertem Alginat (alginat dialdehyde, ADA), Gelatine (Gel), Kollagen, Polymilchsäure, und (gegebenenfalls quervernetzten) Mischungen von zwei oder mehr dieser Polymere. Beispielhaft zu nennen ist hier ADA-Gel, welches oxidiertes Alginat und Gelatine enthält, welche über Reaktion von Alginatdialdehyd mit Aminogruppen der Gelatine miteinander quervernetzt sind.

Beispielsweise werden mittels einer ersten Biotinte Schrittmacherzellen beispielsweise in einem ADA-Gel 1, welches optional weiterhin Fibroblasten enthält (Pacing Core) zu einer dreidimensionalen Struktur ausgeformt; während simultan mittels einer zweiten Biotinte ein zweites ADA-Gel 2 enthaltend Endothelzellen eingebracht wird, sowie ebenfalls simultan mittels einer dritten Biotinte ein drittes ADA-Gel 3, welches Adipozyten und Fibroblasten enthält, eingebracht wird. Insbesondere sofern für die Aufbringung ein FRESH-Verfahren verwendet wird bzw. für die Herstellung von Hohlräumen, beispielsweise innerhalb des Mikrokanalnetzwerkes ein Gelatine-haltiges oder Poloxamer-haltiges Stützmaterial eingesetzt wird (Druck in (thermo)reversibles Stützbad), wird bevorzugt ergänzend eine Quervernetzung, beispielsweise ein ionisches Crosslinking durch Ca²⁺-Ionen und/oder enzymatisches Crosslinking, beispielsweise mit Enzymen aus der Familie der Transglutaminasen, vorzugsweise mikrobielle Transglutaminase, ausgeführt.

In bevorzugten Ausführungsformen des Verfahrens zur Herstellung eines Herzschrittmacher-Konstrukts umfasst dieses weiterhin:
(d) Kultivierung der gemäß (b) erhaltene dreidimensionale Struktur bzw. der gemäß (c) erhaltenen isolierend beschichteten dreidimensionale Struktur
unter Erhalt eines Herzschrittmacher-Konstrukts.

In bevorzugten Ausführungsformen des Verfahrens zur Herstellung eines Herzschrittmacher-Konstrukts erfolgt die Kultivierung gemäß (d) in einem Zellkultur-Medium, weiter bevorzugt bei einer Temperatur im Bereich von 32 bis 41 °C, weiter bevorzugt unter Durchmischung.

In alternativen bevorzugten Ausführungsformen des Verfahrens zur Herstellung eines Herzschrittmacher-Konstrukts, insbesondere eines Herzschrittmacher-Konstrukt gemäß des ersten Aspektes, umfasst das Verfahren:
(a) Bereitstellen von Vorläuferzellen für das Herzschrittmacher-Konstrukt;
(b) Erzeugen einer dreidimensionalen Struktur aus den gemäß (a) bereitgestellten Vorläuferzellen mittels eines artifiziellen Fertigungsverfahrens und/oder eines Abformverfahrens.

In diesen alternativen bevorzugten Ausführungsformen des Verfahrens zur Herstellung eines Herzschrittmacher-Konstrukts werden bevorzugt als Vorläuferzellen Myokardzellen oder humane multipotente Stammzellen oder tierische multipotente Stammzellen oder humane nicht-embryonale pluripotente Stammzellen oder tierische embryonale pluripotente Stammzellen oder tierische nicht-embryonale pluripotente Stammzellen oder tierische parthenogenetische pluripotente Stammzellen oder humane parthenogenetische pluripotente Stammzellen oder tierische spermatogoniale pluripotente Stammzellen oder humane spermatogoniale pluripotente Stammzellen verwendet.

In diesen alternativen bevorzugten Ausführungsformen des Verfahrens zur Herstellung eines Herzschrittmacher-Konstrukts erfolgt die Ausdifferenzierung bevorzugt im ausgeformten Herzschrittmacher-Konstrukt.

In einer möglichen Variante dieser alternativen Ausführungsform werden tierische embryonale Stammzellen als Vorläuferzellen eingesetzt, wobei auch hier die Ausdifferenzierung dann im ausgeformten Herzschrittmacher-Konstrukt erfolgt.

Das Verdrucken von Vorläuferzellen und die anschließende selektive Ausdifferenzierung in Schrittmacherzellen, Fibroblasten, Endothelzellen und Adipozyten, so dass sich die oben beschriebenen Strukturen ausbilden, ist dem Fachmann bekannt. Beispielsweise beschreiben Hofbauer et al. (P. Hofbauer, 2021) und Ergir et al. (Ergir et al., 2022) die Co-Differenzierung in unterschiedliche Zelltypen/Organoide. Das Verdrucken von undifferenzierten hiPSC und anschließender Differenzierung wird beispielsweise von Kupfer et al. beschrieben (Kupfer et al. 2020 ).

Das erfindungsgemäße Herzschrittmacher-Konstrukt ist ausschließlich *ex vivo* bzw. *in vitro* erzeugt. Alle Schritte des Verfahrens zur Herstellung eines Herzschrittmacher-Konstrukts erfolgen entsprechend ausschließlich *ex vivo* bzw. *in vitro.*

### 3. Aspekt - Herzschrittmacher-Konstrukt, erhalten oder erhältlich nach dem Verfahren des zweiten Aspektes

Ein dritter Aspekt der Erfindung betrifft ein Herzschrittmacher-Konstrukt, erhalten oder erhältlich nach dem Verfahren gemäß des zweiten Aspektes, bevorzugt erhalten oder erhältlich nach dem Verfahren gemäß den bevorzugten Ausführungsformen des zweiten Aspektes oder nach dem Verfahren gemäß den alternativen bevorzugten Ausführungsformen des zweiten Aspektes. Alle zum ersten und zum zweiten Aspekt der Erfindung beschriebenen Details, Ausführungsformen und bevorzugten Ausführungsformen gelten gleichfalls für das Herzschrittmacher-Konstrukt des dritten Aspektes.

### 4. Aspekt - Verwendung des Herzschrittmacher-Konstrukts

Ein vierter Aspekt der vorliegenden Erfindung betrifft die Verwendung eines Herzschrittmacher-Konstrukts gemäß des ersten Aspektes oder eines Herzschrittmacher-Konstrukts gemäß des dritten Aspektes, zur Untersuchung der Auswirkung von Stoffen, insbesondere Schadstoffen und/oder Medikamenten, auf die Funktion des sinoatrialen Knotens. Alle zum ersten, zum zweiten und zum dritten Aspekt der Erfindung beschriebenen Details, Ausführungsformen und bevorzugten Ausführungsformen gelten gleichfalls für die Verwendung des vierten Aspektes. Bevorzugt erfolgt die Verwendung *ex vivo* bzw. *in vitro.*

Die vorliegende Erfindung bezieht sich auch auf ein Verfahren zur Behandlung oder Vorbeugung von Krankheiten, vorzugsweise Krankheiten basierend auf einer Fehlfunktion des SAN-Knotens, weiter bevorzugt von Herzrhythmusstörungen, bei einem Patienten, das die Implantation eines Herzschrittmacher-Konstrukts (als Implantat) gemäß der vorliegenden Erfindung in den Patienten umfasst.

Das Behandlungsverfahren der vorliegenden Erfindung ist vorzugsweise ein *in vivo*-Verfahren. Darüber hinaus kann es weitere Schritte zusätzlich zu den oben ausdrücklich genannten umfassen. Weitere Schritte können sich beispielsweise auf die Diagnose der Krankheit vor der Behandlung oder auf die Überwachung des Implantats und/oder des Patienten während der Behandlung beziehen. Darüber hinaus können einer oder mehrere dieser Schritte durch eine automatisierte Ausrüstung durchgeführt werden.

Der Patient bzw. das Subjekt ist ein Säugetier, vorzugsweise ein Mensch.

Der Begriff "Behandlung" bezieht sich auf eine signifikante Verbesserung der hier genannten Krankheiten oder Störungen oder der damit einhergehenden Symptome. Die hier verwendete Bezeichnung "Behandlung" umfasst auch die vollständige Wiederherstellung der Gesundheit im Hinblick auf die hier genannten Krankheiten oder Störungen. Es versteht sich, dass die Behandlung, wie sie hier verwendet wird, nicht bei allen zu behandelnden Personen wirksam sein kann. Der Begriff setzt jedoch voraus, dass vorzugsweise ein statistisch signifikanter Anteil der Personen, die an einer hierin genannten Krankheit oder Störung leiden, erfolgreich behandelt werden kann. Ob ein Anteil statistisch signifikant ist, kann vom Fachmann ohne weiteres mit Hilfe verschiedener bekannter statistischer Auswertungsinstrumente bestimmt werden, beispielsweise Bestimmung von Konfidenzintervallen, Bestimmung des p-Wertes, Student's t-Test, Mann-Whitney-Test usw. Bevorzugte Konfidenzintervalle sind mindestens 90%, mindestens 95%, mindestens 97%, mindestens 98% oder mindestens 99%. Die p-Werte sind vorzugsweise 0,1, 0,05, 0,01, 0,005 oder 0,0001. Vorzugsweise ist die Behandlung bei mindestens 10 %, mindestens 20 %, mindestens 50 %, mindestens 60 %, mindestens 70 %, mindestens 80 % oder mindestens 90 % der Personen einer bestimmten Kohorte oder Population wirksam. Vorzugsweise ist die Behandlung von Krankheiten basierend auf einer Fehlfunktion des SAN-Knotens, weiter bevorzugt von Herzrhythmusstörungen.

Der Begriff "Vorbeugung", wie er hier verwendet wird, bezieht sich auf die Aufrechterhaltung der Gesundheit in Bezug auf die hierin erwähnten Krankheiten oder Störungen für eine bestimmte Zeitspanne bei einem Subjekt. Es versteht sich, dass der genannte Zeitraum von Faktoren wie der Menge verabreichter Arzneimittel und von individuellen Faktoren des Subjekts abhängen kann. Es versteht sich, dass die Vorbeugung nicht bei allen Personen, die mit der erfindungsgemäßen Verbindung behandelt werden, wirksam sein kann. Der Begriff erfordert jedoch, dass vorzugsweise ein statistisch signifikanter Anteil der Subjekte einer Kohorte oder Population wirksam daran gehindert wird, eine Krankheit basierend auf einer Fehlfunktion des SAN-Knotens, weiter bevorzugt eine Herzrhythmusstörung, zu erleiden. Ob ein Anteil statistisch signifikant ist, kann der Fachmann mit Hilfe verschiedener bekannter statistischer Auswertungswerkzeuge, die an anderer Stelle in dieser Beschreibung erläutert werden, ohne weiteres feststellen.

Der Begriff "Implantat" bezeichnet eine Vorrichtung oder ein Material, welche/welches das erfindungsgemäße Herzschrittmacher-Konstrukt umfasst, und welches SAN-Gewebe ganz oder teilweise ersetzt, dass durch eine Krankheit oder einen angeborenen Zustand verloren gegangen sein kann, und welches die normale(n) Funktion(en) des SAN-Knotens wiederherstellen soll. Das Implantat ist bevorzugt biokompatibel, d.h. das Implantat, wenn es in einen Patienten implantiert wird, verursacht keine nachteiligen Wirkungen, wie beispielsweise Toxizität, oder Fremdkörperreaktion.

Die vorliegende Erfindung wird durch die folgende Reihe von Ausführungsformen und Kombinationen von Ausführungsformen, die sich aus den angegebenen Abhängigkeiten und Rückbezügen ergeben, näher erläutert. Insbesondere wurde darauf hingewiesen, dass in jedem Fall, in dem eine Reihe von Ausführungsformen erwähnt wurde, z. B. im Zusammenhang mit einem Begriff wie "eine der Ausführungsformen (1) bis (4)", jede Ausführungsform in dieser Reihe für den Fachmann ausdrücklich offenbart werden sollte, d. h. der Wortlaut dieses Begriffs sollte vom Fachmann als Synonym für "eine der Ausführungsformen (1), (2), (3) und (4)" verstanden werden. Ferner wurde ausdrücklich darauf hingewiesen, dass die folgende Reihe von Ausführungsformen nicht die den Schutzumfang bestimmende Anspruchsgruppe ist, sondern einen geeignet strukturierten Teil der Beschreibung darstellt, der auf allgemeine und bevorzugte Aspekte der vorliegenden Erfindung gerichtet ist.
1. Herzschrittmacher-Konstrukt umfassend eine dreidimensionale Kernstruktur umfassend Schrittmacherzellen und Fibroblasten, *in vitro* hergestellt oder *in vitro* herstellbar mittels eines additiven Fertigungsverfahrens und/oder eines Abformverfahrens.
2. Herzschrittmacher-Konstrukt nach Ausführungsform 1, wobei die Kernstruktur weiterhin Teile der extrazellularen Matrix der Fibroblasten enthält.
3. Herzschrittmacher-Konstrukt nach einer der Ausführungsformen 1 oder 2, wobei innerhalb der Kernstruktur keine nicht-leitfähigen Polymere (wie beispielsweise Polystyrol und/oder Parylen) vorhanden sind, bevorzugt sind innerhalb der Kernstruktur keine Stützelemente aus einem oder mehreren Material(ien) ausgewählt aus der Gruppe von nicht-leitfähigem Kunststoff, Glas, Metall und Metalllegierung, weiter bevorzugt keine Stützelemente nicht-biologischen Ursprungs, vorhanden, wobei weiter bevorzugt generell keine weiteren Stützelemente vorhanden sind.]
4. Herzschrittmacher-Konstrukt nach einer der Ausführungsformen 1 bis 3, wobei die Kernstruktur ein zellbasiertes Verhältnis von Schrittmacherzellen zu Fibroblasten im Bereich von 10:1bis 1:10 aufweist.
5. Herzschrittmacher-Konstrukt nach einer der Ausführungsformen 1 bis 4, wobei die Kernstruktur eine Größe im Bereich von 5 bis 500 mm³, bevorzugt im Bereich von 50 bis 250 mm³ aufweist.
6. Herzschrittmacher-Konstrukt nach einer der Ausführungsformen 1 bis 5, weiterhin umfassend eine Isolationsschicht (fibrotische Schicht) umfassend Adipozyten und Fibroblasten, angeordnet auf der Oberfläche der Kernstruktur, welche die Kernstruktur zu mindestens 90%, bevorzugt zu mindestens 95%, flächendeckend umgibt, wobei ein erster lokal begrenzter Bereich der Oberfläche der Kernstruktur unbedeckt ist, welcher als Signalübertragungsstelle dient oder dienen kann und ein zweiter lokal begrenzter Bereich der Oberfläche der Kernstruktur unbedeckt ist, welcher dem Anschluss bzw. dem Durchtritt eines Blutgefäßes, insbesondere einer Arterie, dient oder dienen kann.
7. Herzschrittmacher-Konstrukt nach einer der Ausführungsformen 1 bis 6, wobei die Isolationsschicht eine Dicke im Bereich von 150 bis 250 µm, bevorzugt im Bereich von 100 bis 300 µm, weiter bevorzugt im Bereich von 50 bis 500 µm, weiter bevorzugt im Bereich von 50 bis 1000 µm, aufweist.
8. Herzschrittmacher-Konstrukt nach einer der Ausführungsformen 1 bis 7, weiterhin umfassend ein Blutgefäß, welches an dem zweiten lokal begrenzten unbedeckten Bereich der Oberfläche der Kernstruktur angeordnet oder anordenbar ist.
9. Herzschrittmacher-Konstrukt nach einer der Ausführungsformen 1 bis 8, wobei das additive Fertigungsverfahren ein dreidimensionales additives Fertigungsverfahren, bevorzugt ein extrusionsbasiertes Biodruck-Verfahren, ein Drop-on-Demand Biodruck-Verfahren, ein lithografisches Verfahren, ein mikrofluidisches Biodruck-Verfahren, ein Laser-unterstütztes Biodruck-Verfahren, oder eine Mischung von zwei oder mehr dieser Verfahren ist, wobei das dreidimensionale additive Fertigungsverfahren bevorzugt ein extrusionsbasiertes Biodruck-Verfahren oder ein lithografisches Verfahren, weiter bevorzugt ein extrusionsbasiertes Biodruck-Verfahren, ist; und wobei das Abformverfahren bevorzugt ein Spritzgussverfahren oder ein Mikrospritzgussverfahren ist.
10. Herzschrittmacher-Konstrukt nach einer der Ausführungsformen 1 bis 9, wobei das Gemisch aus Schrittmacherzellen und Fibroblasten suspendiert in einem Hydrogel aus oxidierter Alginat-Gelatine eingesetzt wird, woraus, bevorzugt mittels extrusionsbasiertem Biodruck-Verfahren, eine dreidimensionale Struktur (Scaffold) erzeugt wird.
11. Herzschrittmacher-Konstrukt nach Ausführungsform 10, wobei die dreidimensionale Struktur, optional mit aufgebrachter Isolationsschicht, kultiviert wird, bevorzugt bei einer Temperatur im Bereich von 32 bis 41 °C, weiter bevorzugt in einem flüssigen Medium, für einen Zeitraum von mindestens einem Tag, bevorzugt mindestens 3 Tagen, weiter bevorzugt mindestens 7 Tagen, unter Erhalt der Kernstruktur aus dreidimensional angeordneten Schrittmacherzellen und Fibroblasten, welche optional von der Isolationsschicht umgeben ist.
12. Herzschrittmacher-Konstrukt nach einer der Ausführungsformen 1 bis 11, weiterhin umfassend ein Mikrokanalnetzwerk zumindest innerhalb der Kernstruktur aus dreidimensional angeordneten Schrittmacherzellen und Fibroblasten, wobei die Wandung der Kanäle des Mikrokanalnetzwerks bevorzugt aus Endothelzellen gebildet ist und weiter bevorzugt die Kanäle des Mikrokanalnetzwerks einen inneren Durchmesser im Bereich von 100-500 µm aufweisen.
13. Herzschrittmacher-Konstrukt nach Ausführungsform 12, wobei das Mikrokanalnetzwerk durch für das Bioprinting der dreidimensionalen Struktur eingesetzte Opfertinten oder durch Druck in ein (thermo)reversibles Stützbad enthaltend ein Stützmaterial erzeugt wird oder erzeugbar ist.
14. Herzschrittmacher-Konstrukt nach einer der Ausführungsformen 1 bis 13, wobei die Kernstruktur zu mindestens 90 Gewichts-%, bevorzugt zu mindestens 95 Gewichts-%, weiter bevorzugt zu mindestens 99 Gewichts-% aus Schrittmacherzellen und Fibroblasten und optional Teilen der extrazellularen Matrix der Fibroblasten, sowie optional Endothelzellen, besteht, jeweils bezogen auf ein Gesamtgewicht der Kernstruktur von 100 Gewichts-%.
15. Herzschrittmacher-Konstrukt nach einer der Ausführungsformen 1 bis 14, wobei die Schrittmacherzellen *in vitro* generierte humane Schrittmacherzellen sind.
16. Herzschrittmacher-Konstrukt nach einer der Ausführungsformen 1 bis 15, wobei die Fibroblasten humane Fibroblasten, bevorzugt humane kardiale Fibroblasten, weiter bevorzugt humane kardiale Sinusknoten-Fibroblasten, sind.
17. Herzschrittmacher-Konstrukt nach einer der Ausführungsformen 1 bis 16, wobei die Adipozyten bevorzugt humane Adipozyten sind.
18. Verfahren zur Herstellung eines Herzschrittmacher-Konstrukts, insbesondere eines Herzschrittmacher-Konstrukt nach einer der Ausführungsformen 1 bis 17, umfassend:
   (a) Bereitstellen einer Mischung aus Schrittmacherzellen und Fibroblasten;
   (b) Erzeugen einer dreidimensionalen Struktur aus der gemäß (a) bereitgestellten Mischung und optional Opfertinte oder einem (thermo)reversiblen Stützbad enthaltend ein Stützmaterial mittels eines artifiziellen Fertigungsverfahrens und/oder eines Abformverfahrens.
19. Verfahren zur Herstellung eines Herzschrittmacher-Konstrukts nach Ausführungsformen 18, wobei die Schritte (a) und (b) umfassend:
   (a.1) Bereitstellen einer Mischung aus Schrittmacherzellen und Fibroblasten;
   (a.2) Bereitstellen von Endothelzellen;
   (b) Erzeugen einer dreidimensionalen Struktur aus der gemäß (a.1) bereitgestellten Mischung und optional Opfertinte oder einem (thermo)reversiblen Stützbad enthaltend ein Stützmaterial mit Erzeugen eines Mikrokanalnetzwerkes umfassend Endothelzellen aus den gemäß (a.2) bereitgestellten Endothelzellen, wobei das Mikrokanalnetzwerk innerhalb der dreidimensionalen Struktur erzeugt wird, jeweils mittels eines artifiziellen Fertigungsverfahrens.
20. Verfahren zur Herstellung eines Herzschrittmacher-Konstrukts nach Ausführungsform 18 oder 19 umfassend:
   (c) Aufbringen einer Isolationsschicht (fibrotische Schicht) umfassend Adipozyten und Fibroblasten auf die Oberfläche der gemäß (b) erzeugten dreidimensionalen Struktur mittels eines artifiziellen Fertigungsverfahrens, wobei mindestens 90%, bevorzugt mindestens 95%, der Oberfläche der gemäß (b) erzeugten dreidimensionalen Struktur bedeckt werden, wobei bevorzugt ein erster lokal begrenzter Bereich der Oberfläche der dreidimensionalen Struktur und optional ein zweiter lokal begrenzter Bereich der Oberfläche der dreidimensionalen Struktur unbedeckt gehalten wird, wobei der erste lokal begrenzte, unbedeckte Bereich der Oberfläche der dreidimensionalen Struktur als Signalübertragungsstelle angelegt ist und der optionale zweite lokal begrenzte, unbedeckte Bereich der Oberfläche als Durchtrittsstelle für eine Blutgefäß, insbesondere einer Arterie, angelegt ist;
   unter Erhalt einer isolierend beschichteten dreidimensionalen Struktur.
21. Verfahren zur Herstellung eines Herzschrittmacher-Konstrukts nach einer der Ausführungsformen 18 bis 20, wobei die Schritte (b) und (c) nacheinander oder gleichzeitig erfolgen.
22. Verfahren zur Herstellung eines Herzschrittmacher-Konstrukts nach einer der Ausführungsformen 18 bis 21, umfassend
   (d) Kultivierung der gemäß (b) erhaltene dreidimensionale Struktur bzw. der gemäß (c) erhaltenen isolierend beschichteten dreidimensionale Struktur unter Erhalt eines Herzschrittmacher-Konstrukts.
23. Verfahren zur Herstellung eines Herzschrittmacher-Konstrukts nach Ausführungsform 22, wobei die Kultivierung gemäß (d) in einem Zellkultur-Medium, bevorzugt bei einer Temperatur im Bereich von 32 bis 41 °C, weiter bevorzugt unter Durchmischung, erfolgt.
24. Verfahren zur Herstellung eines Herzschrittmacher-Konstrukts, insbesondere eines Herzschrittmacher-Konstrukt nach einer der Ausführungsformen 1 bis 17, umfassend:
   (a) Bereitstellen von Vorläuferzellen für das Herzschrittmacher-Konstrukt;
   (b) Erzeugen einer dreidimensionalen Struktur aus den gemäß (a) bereitgestellten Vorläuferzellen mittels eines artifiziellen Fertigungsverfahrens und/oder eines Abformverfahrens.
25. Verfahren zur Herstellung eines Herzschrittmacher-Konstrukts nach Ausführungsformen 24, wobei als Vorläuferzellen Myokardzellen oder humane multipotente Stammzellen oder tierische multipotente Stammzellen oder humane nicht-embryonale pluripotente Stammzellen oder tierische embryonale pluripotente Stammzellen oder tierische nicht-embryonale pluripotente Stammzellen oder tierische parthenogenetische pluripotente Stammzellen oder humane parthenogenetische pluripotente Stammzellen oder tierische spermatogoniale pluripotente Stammzellen oder humane spermatogoniale pluripotente Stammzellen verwendet werden.
26. Verfahren zur Herstellung eines Herzschrittmacher-Konstrukts nach Ausführungsformen 24 oder 25, wobei die Ausdifferenzierung im ausgeformten Herzschrittmacher-Konstrukt erfolgt.
27. Herzschrittmacher-Konstrukt, erhalten oder erhältlich nach dem Verfahren gemäß einer der Ausführungsformen 18 bis 23 oder 24 bis 26.
28. Verwendung eines Herzschrittmacher-Konstrukts nach einer der Ausführungsformen 1 bis 17 oder eines Herzschrittmacher-Konstrukts nach Ausführungsform 27, zur Untersuchung der Auswirkung von Stoffen, insbesondere Schadstoffen und/oder Medikamenten, auf die Funktion des sinoatrialen Knotens.

### Beispiele

Dreidimensionale (3D)-Konstrukte mit vollständig charakterisierten, aus murinen embryonalen Stammzellen (mESC) derivierten Schrittmacherzellen, die mit Fibroblasten angereichert wurden, wurden gedruckt. Hierfür wurde eine Zellmischung aus Schrittmacherzellen und Fibroblasten in einem Hydrogel aus oxidierter Alginat-Gelatine (ADA-GEL) suspendiert und mit einem Extrusionsdrucker zu 3D-scaffolds gedruckt:

### Material und Methoden

### Vorkultivierung der Zellen/ direkte Programmierung induzierter sinoatrialer Körperchen (induced sinoatrial bodies, iSABs):

Für die Generierung der Schrittmacherzellen wurden murine Embryonale Stammzellen angelehnt an das Protokoll (Jung et al. 2014) differenziert. Abweichend wurde hier für die Herstellung der embryonalen Körperchen (Embryoid Bodies) auf "Sphericalplate 5D" Kulturplatten der Firma Kugelmeiers ^{®} zurückgegriffen. Parallel dazu wurden Fibroblastenzellen (BJ) in DMEM (Dulbecco's Modified Eagle's Medium, wenig Glukose) mit 10 Volumen-% FCS (Fetales Kälberserum)(Sigma), 100 U/ml Penicillin-Streptomycin (Gibco) und 100µM MEM (Minimal Essential Medium), angereichert mit nicht-essentiellen Aminosäuren, kultiviert. Nach enzymatischer Vereinzelung einiger Schrittmacherzellcluster an Tag 20-23 der Differenzierung, wurden die Fibroblastenzellen mittels Trypsin abgelöst, die Zellzahlen wurden bestimmt und in einem zellzahl-basiertem Verhältnis von 10:1 (Schrittmacherzellen/Cluster zu Fibroblasten) gemischt.

### Herstellung der Biotinte:

Zur Herstellung der Biotinte wurde Alginat-di-aldehyd (ADA) in Ca²⁺ und Mg²⁺ freier phosphatgepufferter Salzlösung (DPBS, Sigma Aldrich) gelöst (0,375g in 5ml DPBS). Im Anschluss wurden ADA und Gelatine (GEL) im Verhältnis von 1:1 (V:V) zu ADA GEL gemischt und für weitere 15-20 min bei 37°C gerührt. Um eine zellbeladen Biotinte herzustellen, wurde im Anschluss das Zellpellet aus Schrittmacherzellen in Form induzierter sinuatrialer Zellkörperchen (iSABs)-Cluster oder iSAB derivierter Einzelzellen und Fibroblasten in der Biotinte resuspendiert, mit einer Zellkonzentration von ca. 6-7×10⁶ Zellen/ml. Die fertige Biotinte wurde in eine Spritze für den Druckprozess überführt und in die beheizbare Druckerplattform gegeben und für 15-30 min dort auf 28° C temperiert.

### Herstellung von Scaffolds/Gitterstrukturen via 3D Bioprinting:

Nach der erfolgten Temperierung wurden Scaffolds in Form einfacher Gitter mit dem Allevi 1 Bioprinter (Allevi3D, Philadelphia, USA) hergestellt. Genutzt wurde ein Temperaturfenster zwischen 25-28°C bei einem Druck von 1,5 bis 2 bar und einer Verfahrgeschwindigkeit von 2mm/s. Für die Herstellung wurde eine 25G Kanüle genutzt. Die Strangdicke der Scaffolds betrug ca. 250 µm und die Kantenlänge der Scaffolds betrug 10x10mm. Scaffolds bestehend aus 3 Lagen wurden hergestellt. An den 3D Druck schloss sich ein Crosslinking Schritt an, welcher die Form der Scaffolds stabilisierte und die Kultivierbarkeit gewährleistete. Genutzt wurde ein duales Crosslinking Protokoll aus einer Kombination von ionischem Crosslinking durch Ca²⁺-Ionen und einem enzymatische Crosslinking Schritt mit mikrobieller Transglutaminase (mTG): 0,25 g mTG in 10 ml 0,1 molarer CaCl₂ Lösung für 10 min. Im Anschluss wurden die Prints in Nährmedium (DMEM) gegeben und in den Brutschrank überführt bzw. der Print im beheizten Mikroskop bewertet. Der Printprozess wurde mit drei Schrittmacherzelldifferenzierungen durchgeführt.

### Kultivierung der Scaffolds:

Die weitere Kultivierung der Scaffolds erfolgte in den Kulturschalen und Standardkultur-Bedingungen 37°C und 5% CO₂ in Differenzierungsmedium bestehend aus IMDM Medium (Iscove's Modified Dulbecco's Medium, PAN- Biotech GmbH/Biochrom AG), 10 Volumen-% standardisiertes fötales bovines Serum (FCS superior, Biochrom AG), 1% Penicillin-Streptomycin, 100µM MEM nicht essentielle Aminosäuren, 450µM 1-Thioglycerol. Alle 2 Tage wurde das Medium gewechselt und die grundlegende Beschaffenheit der Scaffolds beurteilt. Nach 0, 7, 14, 21 und 28 Tagen in Kultur wurden die Konstrukte strukturell und funktionell analysiert.

### Viabilität der verdruckten Zellen:

Für die Bewertung der Viabilität der Zellen wurde zu verschiedenen Zeitpunkten 0 Tage (0d) (nach dem Print), 14 Tage (14d) und 28 Tage (28d) Calcein AM (grün/lebend) und Ethidium homodimer-1 (rot/tot) Färbungen durchgeführt. Im Anschluss wurden die gefärbten Scaffolds mit dem konfokalen Laserscanning-Mikroskop untersucht und z-Stacks angefertigt. Die Z-Stacks wurden in eine Maximum Intensity Projektion (MIP) überführt und die Anzahl der grünen und roten Signale in Visualisierungssoftware ImageJ quantifiziert.

Die Zellviabilität und Proliferation, welche durch den enzymatischen Substratumsatz von Tetrazolium-Salz zu Formazan durch mitochondriale Dehydrogenase photometrisch im WST-1 Assay gemessen wurde, diente ebenso der Quantifizierung.

### Kalziumaktivität:

Für die Analyse der Kalziumaktivität in den Schrittmacherzellen wurden Stücke des Scaffolds an Tag 7 bzw. Tag 21 mit Calbryte 520 AM, einem fluoreszierenden und zellpermeablen Indikator des intrazellulären Kalziums, angefärbt. Nach einer Inkubationszeit von einer Stunde wurden die Zellen bei 37 °C mit dem konfokalen Laserscanning Mikroskop analysiert. Die Fluoreszenzintensitätspeaks, welche durch die Akkumulation des intrazellulären Kalziums zustande kommen, wurden dann anschließend quantifiziert und mittels eines Scripts in R ausgewertet.

### Bestimmung der Kontraktionsfrequenz:

Die Kontraktionsfrequenz wurde als charakteristischer Marker für die Schrittmacherzellen über die Zeit der Kultivierung ausgewertet. Hierfür wurden Videos von schlagenden Zellen und Zellclustern aufgenommen und in ImageJ mit dem Myocyter Macro¹⁵ ausgewertet. Über das Tool ließ sich eine Schlagfrequenz und Amplitude ermitteln. Für die Auswertung wurde ein abgewandeltes Scaffold Design verwendet, um gezielt Zellen oder Cluster über den gesamten Kultivierungszeitraum von 4 Wochen hinweg analysieren zu können.

### Struktur Bildgebung mittels Second Harmonic Generation - Multiphoton Imaging (SHG-MP):

Um die Mikromorphologie und Differenzierung verdruckter Zellcluster und Schrittmacherzellen bewerten zu können, wurde Second Harmonic Generation Imaging angewandt. Diese Mikroskopietechnik erlaubt die ungelabelte Darstellung nicht-zentrosymmetrischer Strukturen wie Kollagen oder Myosin, welche Aufschluss über die Bildung von actin-myosin Strukturen ermöglicht. (Zitat 10.1364/BOE.4.002078, https://www.nature.com/articles/s41377-018-0080-3, https://opg.optica.org/ol/fulltext.cfm?uri=ol-29-17-2031&id=80997#ref3) SHG Imaging wurde am Ende des Kultivierungszeitraumes an Proben der Scaffolds durchgeführt, welche zuvor mit 4% PFA fixiert wurden.

### Immunhistochemie:

Nach einer Fixierung der Proben mit 4 Volumen-% PFA (Paraformaldehyd), einer Permeabilisierung der Zellen mittels Triton X und dem Blocken unspezifischer Bindungsstellen über eine Serumhaltige Blocklösung wurden mit Antikörpern Zelltyp-spezifische Markerproteine angefärbt: Schrittmacherzellen (Aktinin des glatten Muskels, kardiales TroponinT, Connexin 45, HCN4), Fibroblasten (Vimentin). Außerdem war die Ablagerung zelleigener extrazellulärer Matrix (ECM) von großem Interesse und wurde mittels Färbung von Kollagen 4 untersucht. Es wurde mit primären und passenden sekundären Antikörpern gearbeitet. Die mikroskopische Visualisierung wurde am hochauflösenden konfokalen Laserscanning Mikroskop (LSM 780 ELYRA PS.1) durchgeführt.

### Zellmaterial-Interaktion:

Rasterlektronenmikroskopische (REM) und Transmissionselektronenmikroskopische (TEM) Aufnahmen erfolgten an 3D gedruckten, kultivierten und im Anschluss fixierten (spezifische Fixierungslösung) und gefriergetrockneten Scaffolds.

### Ergebnisse und Diskussion

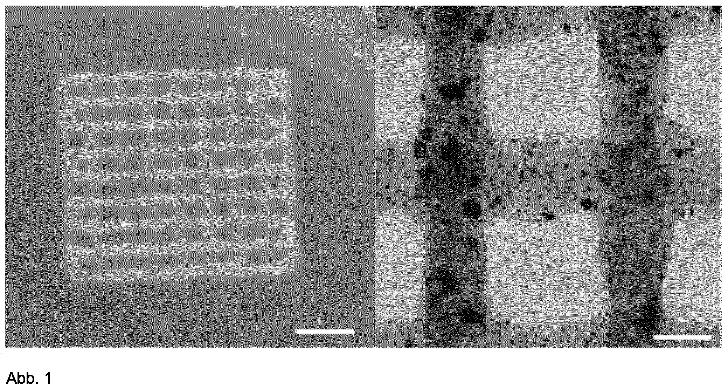

Gezeigt wurde erstmalig die Verdruckbarkeit von Mausschrittmacherzellen (iSAB's)/ Schrittmacherzellclustern gemischt mit Fibroblasten und die Überführung und Kultivierung dieser in Form von gitterförmigen Scaffolds (Abb. 1).

Die Viabilität nach dem Druck war zwar deutlich reduziert, nichtsdestotrotz war eine Kultivierbarkeit und Funktionalität bis zu 28 Tage gewährleistet. Viabilitäten von 50-60% sind typisch für den extrusionsbasierten 3D-Druck, da die Zellen die Überführung in andere Biomaterialien, die Störung/Veränderung der Kulturbedingungen oder auch die starken Scherkräfte während des Druckprozesses nicht in jedem Fall tolerieren. Dies ist jedoch stark abhängig von der genutzten Zelllinie.

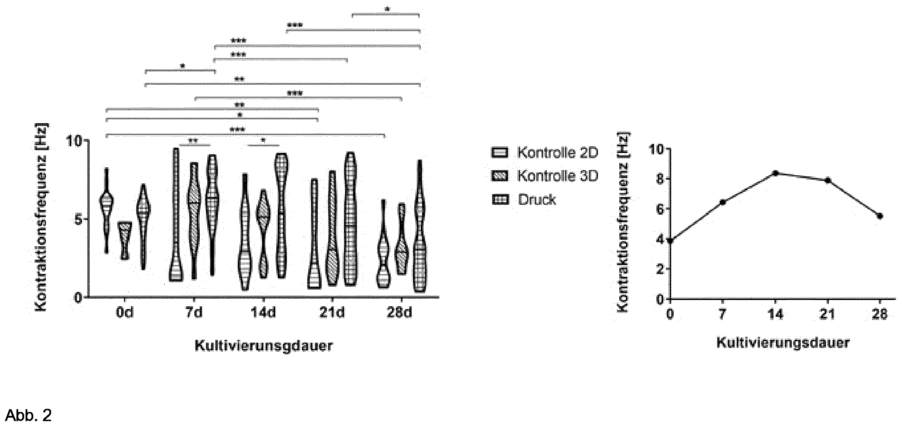

Die Scaffolds konnten bis zu 28 Tage (28d) kultiviert werden und auch bis zu 28d konnten funktionale iSAB's/iSAB-Cluster via Ca²⁺-Imaging und Mikroskopie mit Frequenzanalyse nachgewiesen werden (Video plus CA+ Image). Dies konnte bisher nicht für SAN Schrittmacherzellen gezeigt werden. Die Frequenzen bewegten sich im physiologischen Bereich für alle Vergleichsgruppen, wobei die Frequenz mit Kultivierungsdauer abnahm (Fibroblastenkonkurrenz, Störung Zell-Zell-Kontakte, Konkurrenz um nutritive Versorgung). Die Ergebnisse sind graphisch in Abb. 2 dargestellt.

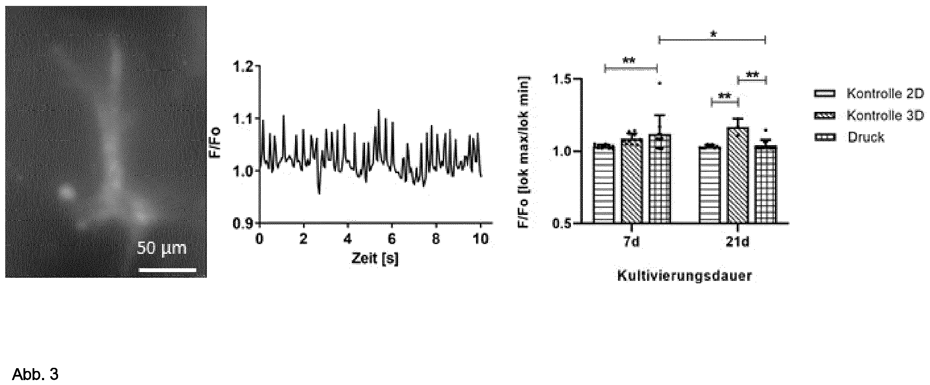

Durch die Visualisierung des intrazellulären Kalziums mittels Calbryte 520 AM, einem fluoreszierenden und zellpermeablen Indikator des intrazellulären Kalziums, konnten die typischen Schwankungen der intrazellulären Kalziumkonzentration während der spontanen Aktivität beobachtet werden, welche die Funktionalität der Schrittmacherzellen belegen. Kalzium ist essentiell im Aktionspotenzial der Schrittmacherzellen und bei der Kopplung von Erregung und Kontraktion. In Abbildung 3 ist eine beispielhafte, spindelförmige Schrittmacherzelle mit einer kurzzeitigen Akkumulation von Kalzium gezeigt, welches durch das starke Fluoreszenzsignal messbar wurde. Darüber hinaus waren die Schwankungen der Konzentration rhythmisch wiederkehrend.

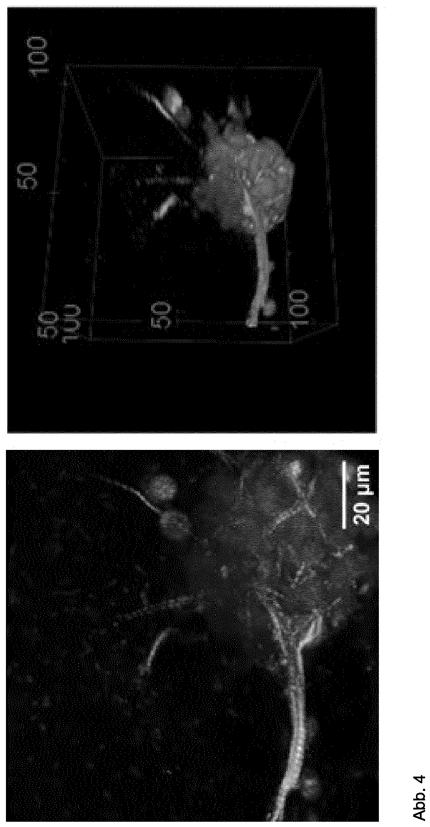

Ein weiteres wichtiges Ergebnis konnte im Multiphotonen Imaging sichtbar gemacht werden. Die Probe zeigte nach einer Kultivierungszeit von 28d geordnete Sarkomerstrukturen, welche durch die typische Struktur von Aktin- und Myosinfilamenten gekennzeichnet waren, die durch Z-Streifen getrennt waren. Sarkomere stellen ein wichtiges Anzeichen für die kontraktile Funktionalität der gedruckten Zellen und Zellcluster dar. Die entsprechenden Bilder sind in Abb. 4 gezeigt.

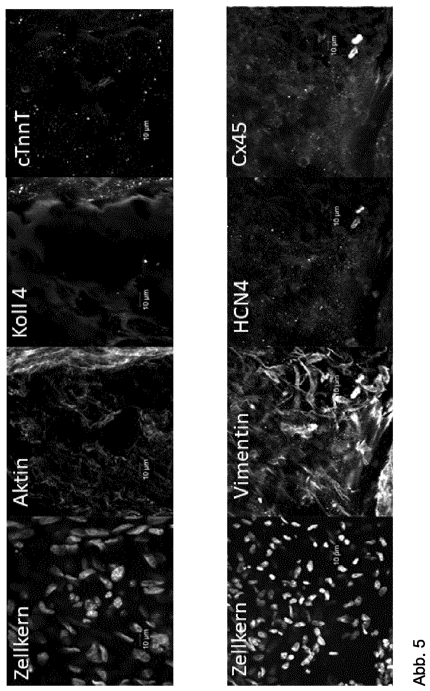

Neben den Sarkomerstrukturen gab es noch weitere Zelltyp-spezifische Markerproteine, welche mit immunhistochemischen Färbungen auf Proteinebene nachgewiesen werden konnten (Abb. 5). Grundsätzlich konnten folgende Makrerproteine nachgewiesen werden: Schrittmacherzellspezifische Expression von Cardiac Troponin (cTnnT), einem Strukturprotein, welches neben Tnnl und TnnC and der Regulation der Muskelkontraktion beteilig ist und im Komplex mit Tropomyosin die Bindung von Aktin und Myosin in Abwesenheit von Kalzium blockiert. Auch das Gap-Junction Protein Connexin 45 ist hauptsächlich in Schrittmacherzellen zu finden und für die Interzelluläre Kommunikation sowie Signalweiterleitung notwendig. In der Signalentstehung des Schrittmacheraktionspotenzials spielt der spezifische lonenkanal HCN4 (Hyperpolarisations-aktivierter zyklischer Nucleotid-gesteuerter Kanal 4, "funny channel") eine essentielle Rolle und ist Hauptbestandteil der Automatizität. Troponin-positive Schrittmacherzellcluster wiesen sowohl Zellausstülpungen als auch teilweise spindelförmige Einzelzellen auf, während die Vimentin-exprimierenden Fibroblasten eine typische längliche Morphologie zeigten. Vimentin dient als Intermediärfilament der Stabilisierung des Cytoskeletts und es somit notwendig während der Proliferation und Migration der Fibroblasten. Letztere sind u.A. an der Bildung von Extrazellulärer Matrix beteiligt. Collagen ist ein Hauptbestandteil dieser Matrix und an der Ausbildung von Zellnetzwerken beteiligt. Die Isoform 4 findet sich hauptsächlich in der Basallamina.

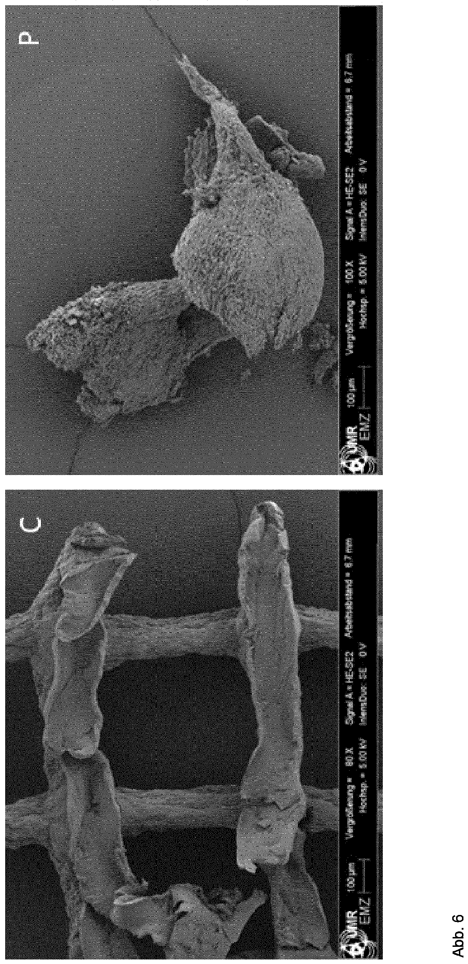

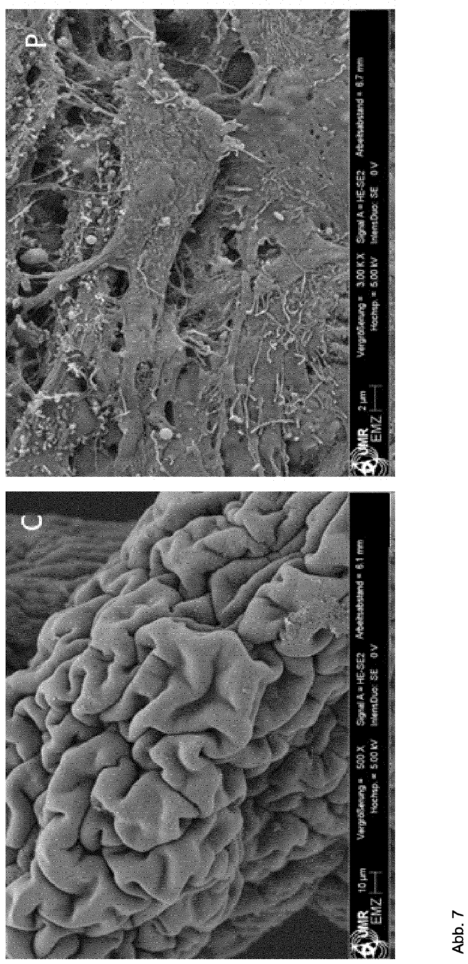

Elektronenmikroskopische Aufnahmen dienten der Analyse von Zell-Zell und Zell-Material Interaktionen. Die Rasterelektronenmikroskopischen Abbildungen (siehe Abb. 6) erlaubten eine detaillierte Analyse der Interaktion auf der Scaffold Oberfläche bzw. aufgebrochenen Strukturen. Hier war in der Kontrolle ein Schrumpfen der Probe sichtbar, welche durch eine dehydrierende Probenvorbereitung zustande kam. Die Zellen breiteten sich in der gedruckten Probe flächig aus und zeigten viele Zellausläufer, dieses sprach für eine gute Zellverträglichkeit des Hydrogels und entsprechende Oberflächenproteine für die Zelladhäsion. Ergänzend dazu ermöglichte die Transmissionselektronenmikroskopie eine Analyse auf Zellebene innerhalb der Probe und kam ohne dehydrierende Behandlungen aus. Die Zellen wiesen typische Zellorganellen auf und zeigten Interzelluläre Verbindungsstellen, die für die Kommunikation, Signalweiterleitung und Adhärenz eine entscheidende Rolle spielen.

Zusammenfassend konnte gezeigt werden, dass hochspezialisierte Herzzellen wie die Schrittmacherzellen des Sinusknotens erfolgreich mit ADA-GEL-Hydrogel 3D-gedruckt werden konnten.

### Kurze Beschreibung der Abbildungen

| | |
|---|---|
| Abb. 1 | zeigt 3D gedrucktes und zellbeladenes ADA-Gel Scaffold in Zellkultur. Skalierungsbalken 2 mm bzw. 200 µm. |
| Abb. 2 | zeigt links ermittelte Frequenzen für 3D-gedruckte Scaffolds, sowie ungedruckte ADA-Gel Referenz (Kontrolle 3D), als auch Matrigel Referenz (Kontrolle 2D). Frequenzen im Rahmen des physiologisch erwartbaren. *p < 0.05; **P < 0.01, ***p < 0.001. Rechts ist gezeigt der Verlauf der Schlagfrequenz eins spezifischen Clusters im gedruckten Scaffold über die gesamte Kultivierungsdauer. |
| Abb.3 | zeigt die rhythmische Akkumulation von Kalzium in den Schrittmacherzellen. Links) spindelförmige Schrittmacherzelle mit hohem intrazellulären Kalzium in gedrucktem Scaffold, Mitte) Messung des Kalziumfluxes innerhalb von 10 Sekunden in einer Zelle/Cluster in gedrucktem Scaffold. Rechts) Mittlerer Kalziumflux dargestellt für alle Conditionen nach 7 und 21 Tagen in Kultur. |
| Abb. 4 | zeigt das SHG-MP Bild eines iSAB-Clusters mit geordneten Sarkomerstrukturen, welche sich in das Hydrogel ausdehnen. |
| Abb. 5 | zeigt einen Immunhistochemischen Nachweis Zelltyp-spezifischer Markerproteine: Schrittmacher-Typischer Marker sind cTnnT (cardiales TroponinT), HCN4 (nicht-selektiver Kationenkanal, "funny channel"), Cx45 (Connexin 45); Fibroblasten Marker ist Vimentin sowie das Matrixprotein Koll IV (Kollagen Typ4), allgemein Aktin als Zytoskelett Marker und DAPI für die Zellkerne. |
| Abb. 6, 7, 8 | zeigen Zell-Zell und Zell-Material-Interaktionen. Abb. 6: Rasterelektronenmikroskopische Aufnahmen der gedruckten Scaffolds. C entspricht der Kontrolle ohne Zellen, wohingegen P für die Zellbeladenen gedruckten Scaffolds steht. Eine deutliche Ausbreitung der Zellen auf dem Hydrogel ist ersichtlich in Abb. 7. Abb. zeigt eineTransmissions-elektronenmikroskopische Aufnahme von Zellen innerhalb des gedruckten Scaffolds. Typische Zellorganellen, sowie notwenige Zell-Zell Verbindungen sind sichtbar. |
| Abb. 9 | zeigt schematisch ein erfindungsgemäßes Herzschrittmacher-Konstrukt umfassend eine Kernstruktur (1), welche Schrittmacherzellen (2) und Fibroblasten (3) sowie Teile der extrazellularen Matrix der Fibroblasten umfasst. Innerhalb der Kernstruktur finden sich ein zentrales Blutgefäß (4) und ein Mikrokanalnetzwerk (5). Das Mikrokanalnetzwerk (5) weist dabei Durchtritte durch die Isolationsschicht auf (anteilig skizziert) und ist gebildet durch Abzweigungen aus dem großen Blutgefäß, welche innerhalb der Kernstruktur ausgebreitet sind. Die Kernstruktur ist, zumindest anteilig, von einer Isolationsschicht (6) bedeckt und es existiert eine Signalübertragungsstelle (7), d.h. ein erster lokal begrenzter Bereich der Oberfläche der Kernstruktur ist unbedeckt. Weiterhin ist die Kernstruktur auch in einem zweiten lokal begrenzten Bereich der Oberfläche der Kernstruktur unbedeckt, welcher dem Anschluss bzw. dem Durchtritt des Blutgefäßes (8), insbesondere einer Arterie, dient oder dienen kann. |

### Angeführte Literatur

- WO 2020/028809 A1
- WO 2013/070952 A1
- WO 2015/091157 A1
- Jung, J. J. et al. Programming and Isolation of Highly Pure Physiologically and Pharmacologically Functional Sinus-Nodal Bodies from Pluripotent Stem Cells. Stem Cell Reports 2, 592-605 (2014)
- Bode, J., Fetzer, C., Nehlsen, K., Scinteie, M., Hinrich, B.H., Baiker, A., Piechaczek, C., Benham, C. and Lipps, H.J. (2001). The hitchhiking principle. Optimizing episomal vectors for the use in gene therapy and biotechnology. Int. J. Gene Ther. Mol. Biol. 6: 33-46
- A. Baiker, C. Maercker, C. Piechaczek, S. B. A. Schmidt, J. Bode, C. Benham, H. J. Lipps in Nature Cell Biology, Vol. 2, Seiten182-184 (2000)
- O. Chabanovska, A.-M. Galow, R. David, H. Lemcke in Adv Drug Deliv Rev., Dezember 2021, 179: 114002
- S. Tohyama, F. Hattori, M. Sano, T. Hishiki, Y. Nagahata, T. Matsuura, H. Hashimoto, T. Suzuki, H.Yamashita, Y. Satoh, T. Egashira, T. Seki, N. Muraoka, H. Yamakawa, Y. Ohgino, T.Tanaka, M. Yoichi, S. Yuasa, M. Murata, M. Suematsu, K. Fukuda in Cell Stem Cell. 2013 Jan 3;12(1):127-37
- P. Hoffbauer et al. in Cell 184, 3299-3317, June 10, 2021
- E. Ergir et al., bioRxiv preprint, 2022 doi: https://doi.org/10.1101/2022.03.07.483273
- M. E. Kupfer et al., Circulation Research. 2020;127:207-224. DOI: 10.1161/CIRCRESAHA.119.316155
- J. Zhang et al., Nature Communications (2019) 10:2238, https://doi.org/10.1038/s41467-019-09831-5
- H. Zhang et al, Circulation Research. 2019; 125:552-566. DOI: 10.1161 /CI RCRESAHA.119.315491
- G. Campostrini et al, Nature Protocols, Vol. 16, April 2021: 2213-2256
- Aghadi et al. (2022),
- T. Ahfeldt et al., Nature Cell Biology Vol. 14 Nr. 2, Februar 2012, 209-219
- Karam et al. Cells 2020, 9, 710; doi:10.3390/cells9030710
- T. Mohsen-Kanson et al., Stem Cells 2014;32:1459-1467
- Noor, N. et al. 3D Printing of Personalized Thick and Perfusable Cardiac Patches and Hearts. Adv. Sci. 6, 1900344 (2019)
- T. J. Hinton et al., Science Advances, Vol. 1, No. 9;1:e1500758 23 October 2015, 1-10
- A . Lee et al., Science, Vol. 365, No. 645202 AUG. 2019, 482-487
- L. G. Brunel et al. 2022 Biofabrication 14 032001
- J. Bliley et al. 2022 Biofabrication14 024106
- Colly et al. Langmuir 2021, 37, 14, 4154-4162

## Patentansprüche

1. Herzschrittmacher-Konstrukt umfassend eine dreidimensionale Kernstruktur umfassend Schrittmacherzellen und Fibroblasten, *in vitro* hergestellt oder *in vitro* herstellbar mittels eines additiven Fertigungsverfahrens und/oder eines Abformverfahrens.

2. Herzschrittmacher-Konstrukt nach Anspruch 1, wobei die Kernstruktur weiterhin Teile der extrazellularen Matrix der Fibroblasten enthält.

3. Herzschrittmacher-Konstrukt nach Anspruch 1 oder 2, weiterhin umfassend eine Isolationsschicht umfassend Adipozyten und Fibroblasten, angeordnet auf der Oberfläche der Kernstruktur, welche die Kernstruktur zu mindestens 90%, bevorzugt zu mindestens 95%, flächendeckend umgibt, wobei ein erster lokal begrenzter Bereich der Oberfläche der Kernstruktur unbedeckt ist, welcher als Signalübertragungsstelle dient oder dienen kann und ein zweiter lokal begrenzter Bereich der Oberfläche der Kernstruktur unbedeckt ist, welcher dem Anschluss bzw. dem Durchtritt eines Blutgefäßes, insbesondere einer Arterie, dient oder dienen kann.

4. Herzschrittmacher-Konstrukt nach einem der Ansprüche 1 bis 3, weiterhin umfassend ein Blutgefäß, welches an dem zweiten lokal begrenzten unbedeckten Bereich der Oberfläche der Kernstruktur angeordnet oder anordenbar ist.

5. Herzschrittmacher-Konstrukt nach einem der Ansprüche 1 bis 4, wobei das additive Fertigungsverfahren ein dreidimensionales additives Fertigungsverfahren, bevorzugt ein extrusionsbasiertes Biodruck-Verfahren, ein Drop-on-Demand Biodruck-Verfahren, ein lithografisches Verfahren, ein mikrofluidisches Biodruck-Verfahren, ein Laser-unterstütztes Biodruck-Verfahren, oder eine Mischung von zwei oder mehr dieser Verfahren ist, wobei das dreidimensionale additive Fertigungsverfahren bevorzugt ein extrusionsbasiertes Biodruck-Verfahren oder ein lithografisches Verfahren, weiter bevorzugt ein extrusionsbasiertes Biodruck-Verfahren, ist; und wobei das Abformverfahren bevorzugt ein Spritzgussverfahren oder ein Mikrospritzgussverfahren ist.

6. Herzschrittmacher-Konstrukt nach einem der Ansprüche 1 bis 5, wobei das Gemisch aus Schrittmacherzellen und Fibroblasten suspendiert in einem Hydrogel aus oxidierter Alginat-Gelatine eingesetzt wird, woraus, bevorzugt mittels extrusionsbasiertem Biodruck-Verfahren, eine dreidimensionale Struktur erzeugt wird.

7. Herzschrittmacher-Konstrukt nach einem der Ansprüche 1 bis 6, weiterhin umfassend ein Mikrokanalnetzwerk zumindest innerhalb der Kernstruktur aus dreidimensional angeordneten Schrittmacherzellen und Fibroblasten, wobei die Wandung der Kanäle des Mikrokanalnetzwerks bevorzugt aus Endothelzellen gebildet ist.

8. Herzschrittmacher-Konstrukt nach einem der Ansprüche 1 bis 7, wobei die Kernstruktur zu mindestens 90 Gewichts-%, bevorzugt zu mindestens 95 Gewichts-%, weiter bevorzugt zu mindestens 99 Gewichts-% aus Schrittmacherzellen und Fibroblasten und optional Teilen der extrazellularen Matrix der Fibroblasten, sowie optional Endothelzellen, besteht, jeweils bezogen auf ein Gesamtgewicht der Kernstruktur von 100 Gewichts-%.

9. Herzschrittmacher-Konstrukt nach einem der Ansprüche 1 bis 8, wobei die Schrittmacherzellen *in vitro* generierte humane Schrittmacherzellen sind; und/oder die Fibroblasten humane Fibroblasten sind und/oder die Adipozyten humane Adipozyten sind.

10. Verfahren zur Herstellung eines Herzschrittmacher-Konstrukts, insbesondere eines Herzschrittmacher-Konstrukt nach einem der Ansprüche 1 bis 9, umfassend:
(a) Bereitstellen einer Mischung aus Schrittmacherzellen und Fibroblasten;
(b) Erzeugen einer dreidimensionalen Struktur aus der gemäß (a) bereitgestellten Mischung und optional Opfertinte oder einem (thermo)reversiblen Stützbad enthaltend ein Stützmaterial mittels eines artifiziellen Fertigungsverfahrens und/oder eines Abformverfahrens.

11. Verfahren zur Herstellung eines Herzschrittmacher-Konstrukts nach Anspruch 10, wobei die Schritte (a) und (b) umfassend:
(a.1) Bereitstellen einer Mischung aus Schrittmacherzellen und Fibroblasten;
(a.2) Bereitstellen von Endothelzellen;
(b) Erzeugen einer dreidimensionalen Struktur aus der gemäß (a.1) bereitgestellten Mischung und optional Opfertinte oder einem (thermo)reversiblen Stützbad enthaltend ein Stützmaterial mit Erzeugen eines Mikrokanalnetzwerkes umfassend Endothelzellen aus den gemäß (a.2) bereitgestellten Endothelzellen, wobei das Mikrokanalnetzwerk innerhalb der dreidimensionalen Struktur erzeugt wird, jeweils mittels eines artifiziellen Fertigungsverfahrens.

12. Verfahren zur Herstellung eines Herzschrittmacher-Konstrukts nach Anspruch 10 oder 11 umfassend:
(c) Aufbringen einer Isolationsschicht umfassend Adipozyten und Fibroblasten auf die Oberfläche der gemäß (b) erzeugten dreidimensionalen Struktur mittels eines artifiziellen Fertigungsverfahrens, wobei mindestens 90%, bevorzugt mindestens 95%, der Oberfläche der gemäß (b) erzeugten dreidimensionalen Struktur bedeckt werden, wobei bevorzugt ein erster lokal begrenzter Bereich der Oberfläche der dreidimensionalen Struktur und optional ein zweiter lokal begrenzter Bereich der Oberfläche der dreidimensionalen Struktur unbedeckt gehalten wird, wobei der erste lokal begrenzte, unbedeckte Bereich der Oberfläche der dreidimensionalen Struktur als Signalübertragungsstelle angelegt ist und der optionale zweite lokal begrenzte, unbedeckte Bereich der Oberfläche als Durchtrittsstelle für eine Blutgefäß, insbesondere einer Arterie, angelegt ist; unter Erhalt einer isolierend beschichteten dreidimensionalen Struktur.

13. Verfahren zur Herstellung eines Herzschrittmacher-Konstrukts, insbesondere eines Herzschrittmacher-Konstrukt nach einem der Ansprüche 1 bis 9, umfassend:
(a) Bereitstellen von Vorläuferzellen für das Herzschrittmacher-Konstrukt;
(b) Erzeugen einer dreidimensionalen Struktur aus den gemäß (a) bereitgestellten Vorläuferzellen mittels eines artifiziellen Fertigungsverfahrens und/oder eines Abformverfahrens.

14. Herzschrittmacher-Konstrukt, erhalten oder erhältlich nach dem Verfahren gemäß einem der Ansprüche 10 bis 12 oder 13.

15. Verwendung eines Herzschrittmacher-Konstrukts nach einem der Ansprüche 1 bis 9 oder eines Herzschrittmacher-Konstrukts nach Anspruch 14, zur Untersuchung der Auswirkung von Stoffen, insbesondere Schadstoffen und/oder Medikamenten, auf die Funktion des sinoatrialen Knotens.
